# EUROPEAN PATENT APPLICATION

(11) **EP 1 813 202 A1**
(43) Date of publication of application: **01.08.2007**
(21) Application number: 07250374.1
(22) Date of filing: 30.01.2007
(51) Int. Cl.: A61B 17/072

(54) **Gearing selector for a powered surgical cutting and fastening instrument**

(30) Priority: 31.01.2006 US 343563
(71) Applicant: ETHICON ENDO-SURGERY, INC., Cincinnati, Ohio 45242 (US)
(72) Inventor: Shelton IV, Frederick E., New Vienna, Ohio (US); Swayze, Jeffrey S., Hamilton, Ohio (US); Timperman, Eugene L., Cincinnati, Ohio (US)
(74) Representative: Tunstall, Christopher Stephen

(57) **Abstract**

A powered surgical cutting and fastening instrument includes a drive shaft; a motor; and a gear shifting assembly connected to the drive shaft and the motor. The gear shifting assembly may include at least a first stage gear assembly coupled to the motor and to the drive shaft for operating the gear shifting assembly in a first gear setting; and a gear coupling assembly for selectively coupling at least one additional gear to the drive shaft for operating the gear shifting assembly in a second gear setting.

## Description

### CROSS-REFERENCE TO RELATED APPLICATIONS

The present application is related to the following concurrently-filed U.S. patent applications, which are incorporated herein by reference:
MOTOR-DRIVEN SURGICAL CUTTING AND FASTENING INSTRUMENT WITH USER FEEDBACK SYSTEM Inventors: Frederick E. Shelton, IV, John Ouwerkerk and Jerome R. Morgan (K&LNG 050519/END5687USNP)
MOTOR-DRIVEN SURGICAL CUTTING AND FASTENING INSTRUMENT WITH LOADING FORCE FEEDBACK Inventors: Frederick E. Shelton, IV, John N. Ouwerkerk, Jerome R. Morgan, and Jeffrey S. Swayze (K&LNG 050516/END5692USNP)
MOTOR-DRIVEN SURGICAL CUTTING AND FASTENING INSTRUMENT WITH TACTILE POSITION FEEDBACK Inventors: Frederick E. Shelton, IV, John N. Ouwerkerk, Jerome R. Morgan, and Jeffrey S. Swayze (K&LNG 050515/END5693USNP)
MOTOR-DRIVEN SURGICAL CUTTING AND FASTENING INSTRUMENT WITH ADAPTIVE USER FEEDBACK Inventors: Frederick E. Shelton, IV, John N. Ouwerkerk, and Jerome R. Morgan (K&LNG 050513/END5694USNP)
MOTOR-DRIVEN SURGICAL CUTTING AND FASTENING INSTRUMENT WITH ARTICULATABLE END EFFECTOR Inventors: Frederick E. Shelton, IV and Christoph L. Gillum (K&LNG 050692/END5769USNP)
MOTOR-DRIVEN SURGICAL CUTTING AND FASTENING INSTRUMENT WITH MECHANICAL CLOSURE SYSTEM Inventors: Frederick E. Shelton, IV and Christoph L. Gillum (K&LNG 050693/END5770USNP)
SURGICAL CUTTING AND FASTENING INSTRUMENT WITH CLOSURE TRIGGER LOCKING MECHANISM Inventors: Frederick E. Shelton, IV and Kevin R. Doll (K&LNG 050694/END5771 USNP)
SURGICAL INSTRUMENT HAVING RECORDING CAPABILITIES Inventors: Frederick E. Shelton, IV, John N. Ouwerkerk, and Eugene L. Timperman (K&LNG 050698/END5773USNP)
SURGICAL INSTRUMENT HAVING A REMOVABLE BATTERY Inventors: Frederick E. Shelton, IV, Kevin R. Doll, Jeffrey S. Swayze and Eugene L. Timperman (K&LNG 050699/END5774USNP)
ELECTRONIC LOCKOUTS AND SURGICAL INSTRUMENT INCLUDING SAME Inventors: Jeffrey S. Swayze, Frederick E. Shelton, IV, Kevin R. Doll (K&LNG 050700/END5775USNP)
ENDOSCOPIC SURGICAL INSTRUMENT WITH A HANDLE THAT CAN ARTICULATE WITH RESPECT TO THE SHAFT Inventors: Frederick E. Shelton, IV, Jeffrey S. Swayze, Mark S. Ortiz, and Leslie M. Fugikawa (K&LNG 050701/END5776USNP)
ELECTRO-MECHANICAL SURGICAL CUTTING AND FASTENING INSTRUMENT HAVING A ROTARY FIRING AND CLOSURE SYSTEM WITH PARALLEL CLOSURE AND ANVIL ALIGNMENT COMPONENTS Inventors: Frederick E. Shelton, IV, Stephen J. Balek and Eugene L. Timperman (K&LNG 050702/END5777USNP)
DISPOSABLE STAPLE CARTRIDGE HAVING AN ANVIL WITH TISSUE LOCATOR FOR USE WITH A SURGICAL CUTTING AND FASTENING INSTRUMENT AND MODULAR END EFFECTOR SYSTEM THEREFOR Inventors: Frederick E. Shelton, IV, Michael S. Cropper, Joshua M. Broehl, Ryan S. Crisp, Jamison J. Float, Eugene L. Timperman (K&LNG 050703/END5778USNP)
SURGICAL INSTRUMENT HAVING A FEEDBACK SYSTEM Inventors: Frederick E. Shelton, IV, Jerome R. Morgan, Kevin R. Doll, Jeffrey S. Swayze and Eugene L. Timperman (K&LNG 050705/EDN5780USNP)

### BACKGROUND

The present invention generally concerns endoscopic surgical instruments and, more particularly, motor-driven endoscopic surgical instruments.

Endoscopic surgical instruments are often preferred over traditional open surgical devices since a smaller incision tends to reduce the post-operative recovery time and complications. Consequently, significant development has gone into a range of endoscopic surgical instruments that are suitable for precise placement of a distal end effector at a desired surgical site through a cannula of a trocar. These distal end effectors engage the tissue in a number of ways to achieve a diagnostic or therapeutic effect (e.g., endocutter, grasper, cutter, staplers, clip applier, access device, drug/gene therapy delivery device, and energy device using ultrasound, RF, laser, etc.).

Known surgical staplers include an end effector that simultaneously makes a longitudinal incision in tissue and applies lines of staples on opposing sides of the incision. The end effector includes a pair of cooperating jaw members that, if the instrument is intended for endoscopic or laparoscopic applications, are capable of passing through a cannula passageway. One of the jaw members receives a staple cartridge having at least two laterally spaced rows of staples. The other jaw member defines an anvil having staple-forming pockets aligned with the rows of staples in the cartridge. The instrument includes a plurality of reciprocating wedges which, when driven distally, pass through openings in the staple cartridge and engage drivers supporting the staples to effect the firing of the staples toward the anvil.

An example of a surgical stapler suitable for endoscopic applications is described in U.S. Pat. No. 5,465,895, which discloses an endocutter with distinct closing and firing actions. A clinician using this device is able to close the jaw members upon tissue to position the tissue prior to firing. Once the clinician has determined that the jaw members are properly gripping tissue, the clinician can then fire the surgical stapler with a single firing stroke, thereby severing and stapling of the tissue. The simultaneous severing and stapling avoids complications that may arise when performing such actions sequentially with different surgical tools that respectively only sever or staple.

One specific advantage of being able to close upon tissue before firing is that the clinician is able to verify via an endoscope that the desired location for the cut has been achieved, including a sufficient amount of tissue has been captured between opposing jaws. Otherwise, opposing jaws may be drawn too close together, especially pinching at their distal ends, and thus not effectively forming closed staples in the severed tissue. At the other extreme, an excessive amount of clamped tissue may cause binding and an incomplete firing.

Endoscopic staplers/cutters continue to increase in complexity and function with each generation. One of the main reasons for this is the quest for lower force-to-fire (FTF) to a level that all or a great majority of surgeons can handle. One known solution to lower FTF it use CO₂ or electrical motors. These devices have not faired much better than traditional hand-powered devices, but for a different reason. Surgeons typically prefer to experience proportionate force distribution to that being experienced by the end-effector in the forming the staple to assure them that the cutting/stapling cycle is complete, with the upper limit within the capabilities of most surgeons (usually around 15-30 lbs). They also typically want to maintain control of deploying the staple and being able to stop at anytime if the forces felt in the handle of the device feel too great or for some other clinical reason. These user-feedback effects are not suitably realizable in present motor-driven endocutters. As a result, there is a general lack of acceptance by physicians of motor-drive endocutters where the cutting/stapling operation is actuated by merely pressing a button.

Depending on the type and density of tissue being stapled and cut, more power or more precision may be desired from the surgical stapling and cutting instrument in various situations. For example, if the surgeon needs to staple and cut a relatively dense section of tissue, as could be the case in revisional surgery, it would be helpful for the instrument to be able to adjust the gear setting of the motor to deliver more torque and less speed to accommodate the denser tissue. In general, the ability to adjust gear settings for the instrument would promote increased control of the end-effector, especially when the surgeon operates on various types of exceptionally dense or exceptionally thin tissue.

### SUMMARY

In various embodiments, the invention is directed to a powered surgical cutting and fastening instrument. The one embodiment, the instrument comprises: (a) a drive shaft; (b) a motor; and (c) a gear shifting assembly connected to the drive shaft and the motor, the gear shifting assembly comprising: at least a first stage gear assembly coupled to the motor and to the drive shaft for operating the gear shifting assembly in a first gear setting; and a gear coupling assembly for selectively coupling at least one additional gear to the drive shaft for operating the gear shifting assembly in a second gear setting.

The first stage gear assembly may comprises a sun gear intermeshed at least partially with one or more planet gears to provide a planetary gear arrangement for the first stage gear assembly.

The instrument may further comprise a second stage gear assembly connected to the first stage gear assembly.

At least one of the first stage gear assembly or the second stage gear assembly may comprises a sun gear intermeshed at least partially with one or more planet gears to provide a planetary gear arrangement for the first stage gear assembly or the second stage gear assembly.

The gear coupling assembly may further comprises a sun gear at least partially intermeshed with one or more planet gears to provide a planetary gear arrangement which can be selectively coupled to the first stage gear assembly.

The sun gear of the gear coupling assembly may further include a spline section structured to correspondingly intermesh in the second gear setting with a spline section formed on an input shaft received from the first stage gear assembly into the gear coupling assembly.

The sun gear of the gear coupling assembly may further include a spline section structured to not correspondingly intermesh in the first gear setting with a spline section formed on an input shaft received from the first stage gear assembly into the gear coupling assembly.

The gear coupling assembly may further comprise a collar including a spline section formed therein.

The instrument may further comprise the spline section of the collar being structured to correspondingly intermesh with a spline section formed on the drive shaft in the first gear setting or the second gear setting.

The gear shifting assembly may further comprise a gear selector assembly for moving the gear coupling assembly between the first and second gear settings.

The gear selector assembly may further comprise a switch connected to a yoke operatively associated with a collar of the gear coupling assembly.

The instrument may further comprise at least one bevel gear assembly connected to the motor and to the first stage gear assembly of the gear shifting assembly.

The first stage gear assembly and the second stage gear assembly may each include a sun gear intermeshed at least partially with one or more planet gears to provide planetary gear arrangements for the first stage gear assembly and the second stage gear assembly.

The gear coupling assembly may further comprise a sun gear at least partially intermeshed with one or more planet gears to provide a planetary gear arrangement which can be selectively coupled to the second stage gear assembly.

The sun gear of the gear coupling assembly may further include a spline section structured to correspondingly intermesh in the second gear setting with a spline section formed on an input shaft received into the gear coupling assembly from the second stage gear assembly.

The sun gear of the gear coupling assembly may further include a spline section structured to not correspondingly intermesh in the first gear setting with a spline section formed on an input shaft received into the gear coupling assembly from the second stage gear assembly.

In another embodiment, the present invention discloses a surgical cutting and fastening instrument comprising: (a) a drive shaft; (b) a motor; and (c) a gear shifting assembly connected to the drive shaft and the motor, the gear shifting assembly comprising: a first stage gear assembly coupled to the motor and to the drive shaft for operating the gear shifting assembly in a first gear setting, the first stage gear assembly comprising a sun gear intermeshed at least partially with one or more planet gears to provide a planetary gear arrangement for the first stage gear assembly; a second gear stage assembly connected to the first gear stage assembly and to the drive shaft, the second stage gear assembly comprising a sun gear intermeshed at least partially with one or more planet gears to provide a planetary gear arrangement for the second stage gear assembly; a gear coupling assembly for selectively coupling at least one additional gear to the second stage gear assembly for operating the gear shifting assembly in a second gear setting, the gear coupling assembly further comprising a sun gear at least partially intermeshed with one or more planet gears to provide a planetary gear arrangement which can be selectively coupled to the first stage gear assembly.

The sun gear of the gear coupling assembly may further include a spline section structured to correspondingly intermesh in the second gear setting with a spline section formed on an input shaft received into the gear coupling assembly from the second stage gear assembly.

The sun gear of the gear coupling assembly may further include a spline section structured to not correspondingly intermesh in the first gear setting with a spline section formed on an input shaft received into the gear coupling assembly from the second stage gear assembly.

In another embodiment, the present invention discloses a surgical cutting and fastening instrument comprising: (a) a drive shaft; (b) a motor; and (c) a gear shifting assembly connected to the drive shaft and the motor, the gear shifting assembly comprising: a first stage gear assembly coupled to the motor and to the drive shaft for operating the gear shifting assembly in a first gear setting, the first stage gear assembly comprising a sun gear intermeshed at least partially with one or more planet gears to provide a planetary gear arrangement for the first stage gear assembly; a second gear stage assembly connected to the first gear stage assembly and to the drive shaft, the second stage gear assembly comprising a sun gear intermeshed at least partially with one or more planet gears to provide a planetary gear arrangement for the second stage gear assembly; a gear coupling assembly for selectively coupling at least one additional gear to the second stage gear assembly for operating the gear shifting assembly in a second gear setting, the gear coupling assembly further comprising a sun gear at least partially intermeshed with one or more planet gears to provide a planetary gear arrangement which can be selectively coupled to the second stage gear assembly; and, wherein the sun gear of the gear coupling assembly further includes a spline section structured to correspondingly intermesh in the second gear setting with a spline section formed on an input shaft received into the gear coupling assembly from the second stage gear assembly, and the spline section of the sun gear of the gear coupling assembly further being structured to not correspondingly intermesh in the first gear setting with a spline section formed on an input shaft received into the gear coupling assembly from the second stage gear assembly.

### DRAWINGS

Various embodiments of the present invention are described herein by way of example in conjunction with the following figures, wherein
Figures 1 and 2 are perspective views of an endoscopic surgical instrument according to various embodiments of the present invention;
Figures 3-5 are exploded views of an end effector and shaft of the instrument according to various embodiments of the present invention;
Figure 6 is a side view of the end effector according to various embodiments of the present invention;
Figure 7 is an exploded view of the handle of the instrument according to various embodiments of the present invention;
Figures 8 and 9 are partial perspective views of the handle according to various embodiments of the present invention;
Figure 10 is a side view of the handle according to various embodiments of the present invention;
Figure 11 is a schematic diagram of a circuit used in the instrument according to various embodiments of the present invention;
Figures 12-13 are side views of the handle according to other embodiments of the present invention;
Figures 14-22 illustrate different mechanisms for locking the closure trigger according to various embodiments of the present invention;
Figures 23A-B show a universal joint ("u-joint") that may be employed at the articulation point of the instrument according to various embodiments of the present invention;
Figures 24A-B shows a torsion cable that may be employed at the articulation point of the instrument according to various embodiments of the present invention;
Figures 25-31 illustrate an endoscopic surgical instrument with power assist according to another embodiment of the present invention;
Figures 32-36 illustrate an endoscopic surgical instrument with power assist according to yet another embodiment of the present invention;
Figures 37-40 illustrate an endoscopic surgical instrument with tactile feedback to embodiments of the present invention;
Figures 41-42 illustrate a proportional sensor that may be used according to various embodiments of the present invention;
Figure 43 includes a side view of a handle of a surgical instrument that may be provided in association with embodiments of the invention;
Figure 44 illustrates a partially cross-sectional, partially schematic side view of a gear shifting assembly that can be provided in accordance with embodiments of the invention;
Figure 45 illustrates a schematic of a planetary gear arrangement that can be provided in accordance with embodiments of the invention;
Figure 46 is an enlarged view of a section of Figure 44;
Figure 47 includes an exploded view of a gear shifting assembly that can be provided in accordance with embodiments of the invention;
Figure 48 includes a partially cross-sectional, partially schematic side view of a gear shifting assembly that can be provided in accordance with embodiments of the invention;
Figure 49 is a view of a section taken through Figure 48; and
Figure 50 is an enlarged view of a section of Figure 48.

### DETAILED DESCRIPTION

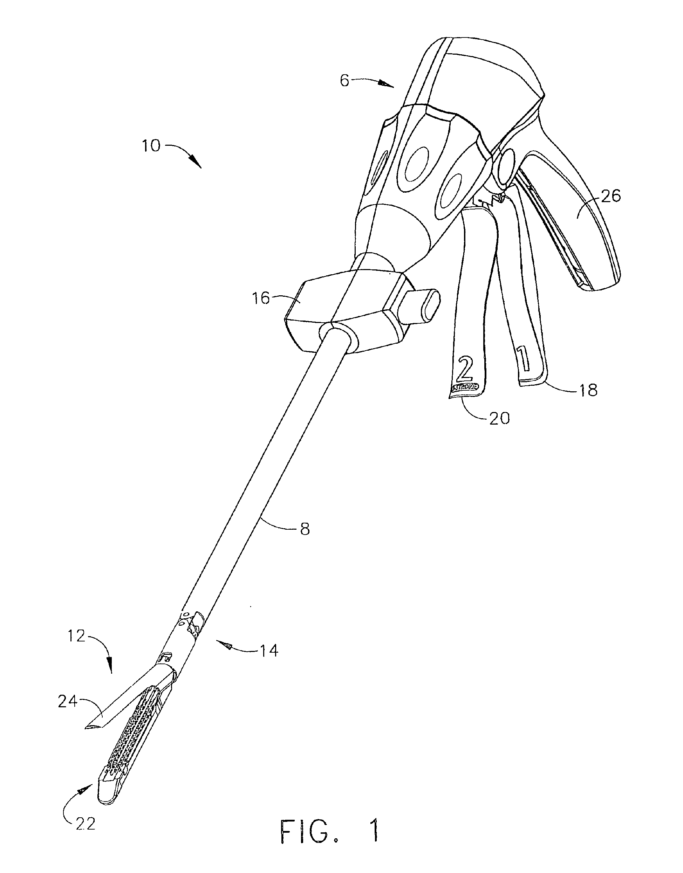

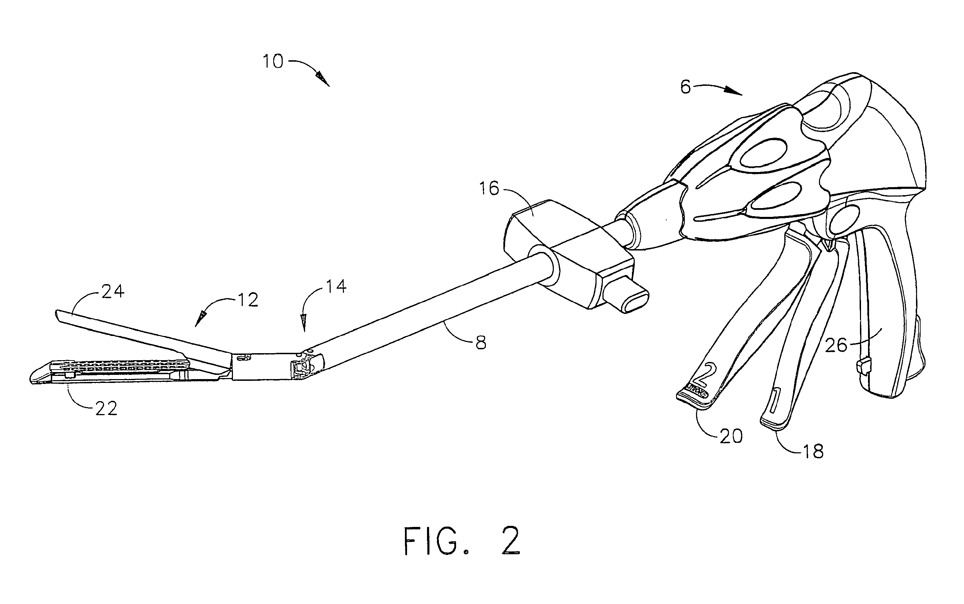

Figures 1 and 2 depict an endoscopic surgical instrument 10 according to various embodiments of the present invention. The endoscopic surgical instrument 10 comprises a handle 6, a shaft 8, and an articulating end effector 12 pivotally connected to the shaft 8 at an articulation pivot 14. An articulation control 16 may be provided adjacent to the handle 6 to effect rotation of the end effector 12 about the articulation pivot 14. In the illustrated embodiment, the end effector 12 is configured to act as an endocutter for clamping, severing and stapling tissue, although, in other embodiments, different types of end effectors may be used, such as end effectors for other types of surgical devices, such as graspers, cutters, staplers, clip appliers, access devices, drug/gene therapy devices, ultrasound, RF or laser devices, etc.

The handle 6 of the instrument 10 may include a closure trigger 18 and a firing trigger 20 for actuating the end effector 12. It will be appreciated that instruments having end effectors directed to different surgical tasks may have different numbers or types of triggers or other suitable controls for operating the end effector 12. The end effector 12 is shown separated from the handle 6 by a preferably elongate shaft 8. In one embodiment, a clinician or operator of the instrument 10 may articulate the end effector 12 relative to the shaft 8 by utilizing the articulation control 16, as described in more detail in pending U.S. patent application Ser. No. 11/329,020, filed January 10, 2006, entitled "Surgical Instrument Having An Articulating End Effector," by Geoffrey C. Hueil et al., which is incorporated herein by reference.

The end effector 12 includes in this example, among other things, a staple channel 22 and a pivotally translatable clamping member, such as an anvil 24, which are maintained at a spacing that assures effective stapling and severing of tissue clamped in the end effector 12. The handle 6 includes a pistol grip 26 towards which a closure trigger 18 is pivotally drawn by the clinician to cause clamping or closing of the anvil 24 toward the staple channel 22 of the end effector 12 to thereby clamp tissue positioned between the anvil 24 and channel 22. The firing trigger 20 is farther outboard of the closure trigger 18. Once the closure trigger 18 is locked in the closure position as further described below, the firing trigger 20 may rotate slightly toward the pistol grip 26 so that it can be reached by the operator using one hand. Then the operator may pivotally draw the firing trigger 20 toward the pistol grip 26 to cause the stapling and severing of clamped tissue in the end effector 12. In other embodiments, different types of clamping members besides the anvil 24 could be used, such as, for example, an opposing jaw, etc.

It will be appreciated that the terms "proximal" and "distal" are used herein with reference to a clinician gripping the handle 6 of an instrument 10. Thus, the end effector 12 is distal with respect to the more proximal handle 6. It will be further appreciated that, for convenience and clarity, spatial terms such as "vertical" and "horizontal" are used herein with respect to the drawings. However, surgical instruments are used in many orientations and positions, and these terms are not intended to be limiting and absolute.

The closure trigger 18 may be actuated first. Once the clinician is satisfied with the positioning of the end effector 12, the clinician may draw back the closure trigger 18 to its fully closed, locked position proximate to the pistol grip 26. The firing trigger 20 may then be actuated. The firing trigger 20 returns to the open position (shown in Figures 1 and 2) when the clinician removes pressure, as described more fully below. A release button on the handle 6, when depressed, may release the locked closure trigger 18.

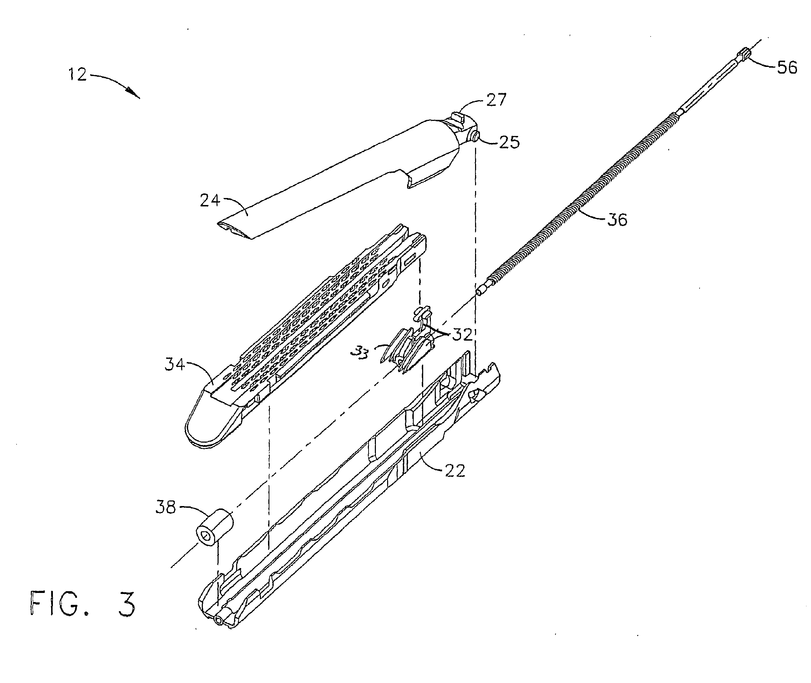

Figure 3 is an exploded view of the end effector 12 according to various embodiments. As shown in the illustrated embodiment, the end effector 12 may include, in addition to the previously-mentioned channel 22 and anvil 24, a cutting instrument 32, a sled 33, a staple cartridge 34 that is removably seated in the channel 22, and a helical screw shaft 36. The cutting instrument 32 may be, for example, a knife. The anvil 24 may be pivotably opened and closed at a pivot point 25 connected to the proximate end of the channel 22. The anvil 24 may also include a tab 27 at its proximate end that is inserted into a component of the mechanical closure system (described further below) to open and close the anvil 24. When the closure trigger 18 is actuated, that is, drawn in by a user of the instrument 10, the anvil 24 may pivot about the pivot point 25 into the clamped or closed position. If clamping of the end effector 12 is satisfactory, the operator may actuate the firing trigger 20, which, as explained in more detail below, causes the knife 32 and sled 33 to travel longitudinally along the channel 22, thereby cutting tissue clamped within the end effector 12. The movement of the sled 33 along the channel 22 causes the staples (not shown) of the staple cartridge 34 to be driven through the severed tissue and against the closed anvil 24, which turns the staples to seal the severed tissue. U.S. Pat. 6,978,921, entitled "Surgical stapling instrument incorporating an E-beam firing mechanism," which is incorporated herein by reference, provides more details about such two-stroke endoscopic instruments. The sled 33 may be part of the cartridge 34, such that when the knife 32 retracts following the cutting operation, the sled 33 does not retract.

It should be noted that although the embodiments of the instrument 10 described herein employ an end effector 12 that staples the severed tissue, in other embodiments different techniques for closing or sealing the severed tissue may be used. For example, end effectors that use RF energy or adhesives to seal the severed tissue may also be used. U.S. Pat. No. 5,688,270 entitled "Electrosurgical Hemostatic Device with Recessed and/or Offset Electrodes" to Yates et al., and U.S. Pat. No. 5,709,680 entitled "Electrosurgical Hemostatic Device" to Yates et al., which are incorporated herein by reference, disclose an endoscopic cutting instrument that uses RF energy to seal the severed tissue. U.S. Patent Application Serial No. 11/267,811 to Jerome R. Morgan, *et. al,* and U.S. Patent Application Serial No. 11/267,383 to Frederick E. Shelton, IV, *et. al*, which are also incorporated herein by reference, disclose an endoscopic cutting instrument that uses adhesives to seal the severed tissue. Accordingly, although the description herein refers to cutting/stapling operations and the like below, it should be recognized that this is an exemplary embodiment and is not meant to be limiting. Other tissue-sealing techniques may also be used.

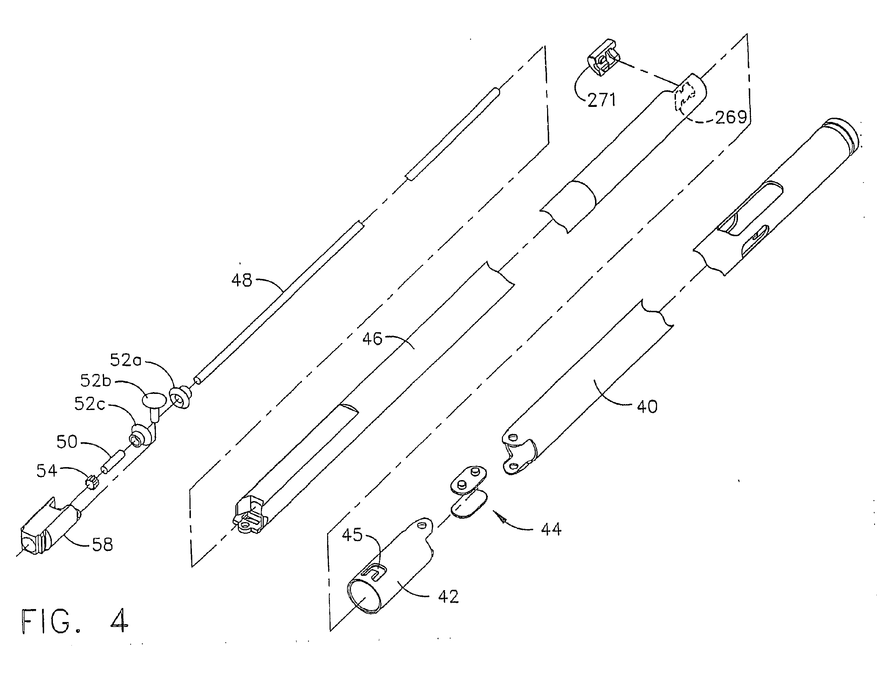

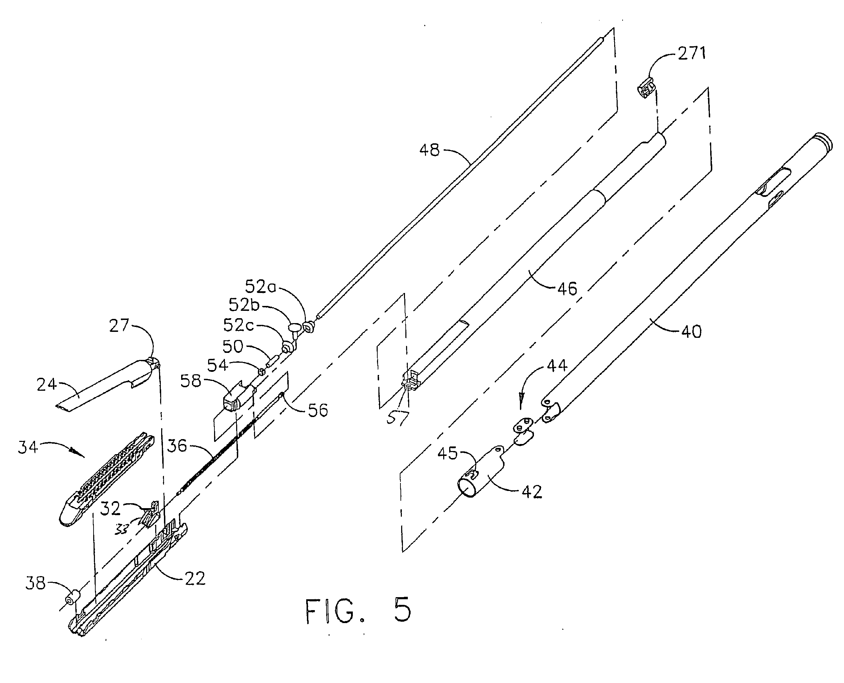

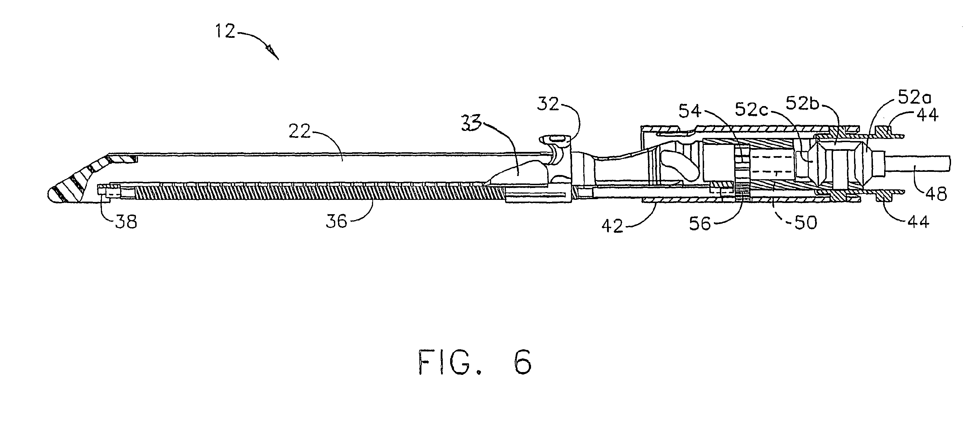

Figures 4 and 5 are exploded views and Figure 6 is a side view of the end effector 12 and shaft 8 according to various embodiments. As shown in the illustrated embodiment, the shaft 8 may include a proximate closure tube 40 and a distal closure tube 42 pivotably linked by a pivot links 44. The distal closure tube 42 includes an opening 45 into which the tab 27 on the anvil 24 is inserted in order to open and close the anvil 24, as further described below. Disposed inside the closure tubes 40, 42 may be a proximate spine tube 46. Disposed inside the proximate spine tube 46 may be a main rotational (or proximate) drive shaft 48 that communicates with a secondary (or distal) drive shaft 50 via a bevel gear assembly 52. The secondary drive shaft 50 is connected to a drive gear 54 that engages a proximate drive gear 56 of the helical screw shaft 36. The vertical bevel gear 52b may sit and pivot in an opening 57 in the distal end of the proximate spine tube 46. A distal spine tube 58 may be used to enclose the secondary drive shaft 50 and the drive gears 54, 56. Collectively, the main drive shaft 48, the secondary drive shaft 50, and the articulation assembly (e.g., the bevel gear assembly 52a-c) are sometimes referred to herein as the "main drive shaft assembly."

The sled 33 may be made of, for example, plastic, and may have a sloped distal surface. As the sled 33 traverses the channel 22, the sloped forward surface may push up or drive the staples in the staple cartridge through the clamped tissue and against the anvil 24. The anvil 24 turns the staples, thereby stapling the severed tissue. When the knife 32 is retracted, the knife 32 and sled 33 may become disengaged, thereby leaving the sled 33 at the distal end of the channel.

As described above, because of the lack of user feedback for the cutting/stapling operation, there is a general lack of acceptance among physicians of motor-driven endocutters where the cutting/stapling operation is actuated by merely pressing a button. In contrast, embodiments of the present invention provide a motor-driven endocutter with user-feedback of the deployment, force, and/or position of the cutting instrument in the end effector.

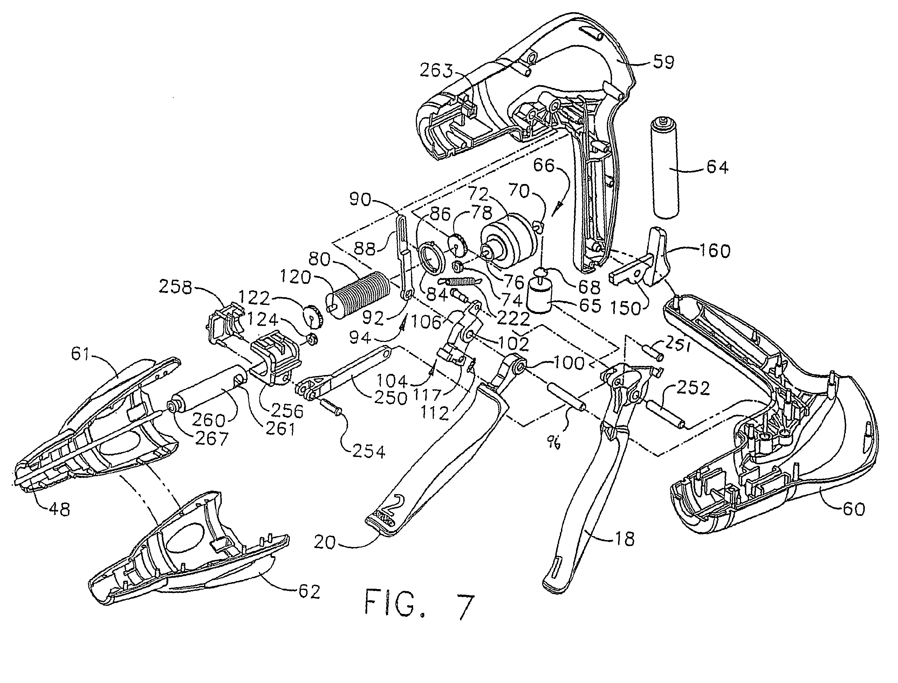

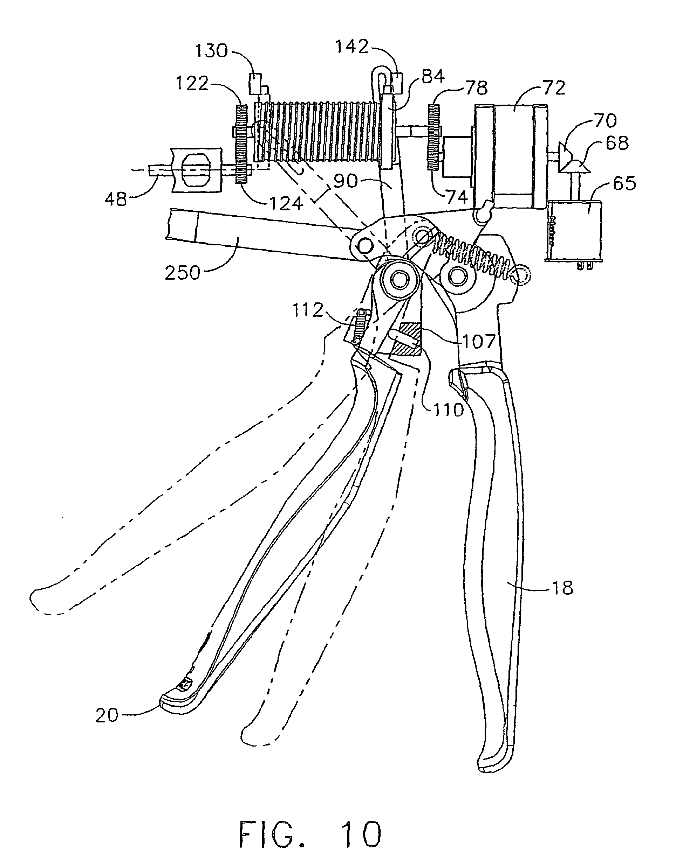

Figures 7-10 illustrate an exemplary embodiment of a motor-driven endocutter, and in particular the handle 6 thereof, that provides user-feedback regarding the deployment and loading force of the cutting instrument in the end effector. In addition, the embodiment may use power provided by the user in retracting the firing trigger 20 to power the device (a so-called "power assist" mode). As shown in the illustrated embodiment, the handle 6 includes exterior lower side pieces 59, 60 and exterior upper side pieces 61, 62 that fit together to form, in general, the exterior of the handle 6. A battery 64, such as a Li ion battery, may be provided in the pistol grip portion 26 of the handle 6. The battery 64 powers a motor 65 disposed in an upper portion of the pistol grip portion 26 of the handle 6. According to various embodiments, the motor 65 may be a DC brushed driving motor having a maximum rotation of, approximately, 5000 RPM. The motor 65 may drive a 90° bevel gear assembly 66 comprising a first bevel gear 68 and a second bevel gear 70. The bevel gear assembly 66 may drive a planetary gear assembly 72. The planetary gear assembly 72 may include a pinion gear 74 connected to a drive shaft 76. The pinion gear 74 may drive a mating ring gear 78 that drives a helical gear drum 80 via a drive shaft 82. A ring 84 may be threaded on the helical gear drum 80. Thus, when the motor 65 rotates, the ring 84 is caused to travel along the helical gear drum 80 by means of the interposed bevel gear assembly 66, planetary gear assembly 72 and ring gear 78.

The handle 6 may also include a run motor sensor 110 in communication with the firing trigger 20 to detect when the firing trigger 20 has been drawn in (or "closed") toward the pistol grip portion 26 of the handle 6 by the operator to thereby actuate the cutting/stapling operation by the end effector 12. The sensor 110 may be a proportional sensor such as, for example, a rheostat or variable resistor. When the firing trigger 20 is drawn in, the sensor 110 detects the movement, and sends an electrical signal indicative of the voltage (or power) to be supplied to the motor 65. When the sensor 110 is a variable resistor or the like, the rotation of the motor 65 may be generally proportional to the amount of movement of the firing trigger 20. That is, if the operator only draws or closes the firing trigger 20 in a little bit, the rotation of the motor 65 is relatively low. When the firing trigger 20 is fully drawn in (or in the fully closed position), the rotation of the motor 65 is at its maximum. In other words, the harder the user pulls on the firing trigger 20, the more voltage is applied to the motor 65, causing greater rates of rotation.

The handle 6 may include a middle handle piece 104 adjacent to the upper portion of the firing trigger 20. The handle 6 also may comprise a bias spring 112 connected between posts on the middle handle piece 104 and the firing trigger 20. The bias spring 112 may bias the firing trigger 20 to its fully open position. In that way, when the operator releases the firing trigger 20, the bias spring 112 will pull the firing trigger 20 to its open position, thereby removing actuation of the sensor 110, thereby stopping rotation of the motor 65. Moreover, by virtue of the bias spring 112, any time a user closes the firing trigger 20, the user will experience resistance to the closing operation, thereby providing the user with feedback as to the amount of rotation exerted by the motor 65. Further, the operator could stop retracting the firing trigger 20 to thereby remove force from the sensor 100, to thereby stop the motor 65. As such, the user may stop the deployment of the end effector 12, thereby providing a measure of control of the cutting/sealing operation to the operator.

The distal end of the helical gear drum 80 includes a distal drive shaft 120 that drives a ring gear 122, which mates with a pinion gear 124. The pinion gear 124 is connected to the main drive shaft 48 of the main drive shaft assembly. In that way, rotation of the motor 65 causes the main drive shaft assembly to rotate, which causes actuation of the end effector 12, as described above.

The ring 84 threaded on the helical gear drum 80 may include a post 86 that is disposed within a slot 88 of a slotted arm 90. The slotted arm 90 has an opening 92 its opposite end 94 that receives a pivot pin 96 that is connected between the handle exterior side pieces 59, 60. The pivot pin 96 is also disposed through an opening 100 in the firing trigger 20 and an opening 102 in the middle handle piece 104.

In addition, the handle 6 may include a reverse motor (or end-of-stroke sensor) 130 and a stop motor (or beginning-of-stroke) sensor 142. In various embodiments, the reverse motor sensor 130 may be a limit switch located at the distal end of the helical gear drum 80 such that the ring 84 threaded on the helical gear drum 80 contacts and trips the reverse motor sensor 130 when the ring 84 reaches the distal end of the helical gear drum 80. The reverse motor sensor 130, when activated, sends a signal to the motor 65 to reverse its rotation direction, thereby withdrawing the knife 32 of the end effector 12 following the cutting operation.

The stop motor sensor 142 may be, for example, a normally-closed limit switch. In various embodiments, it may be located at the proximate end of the helical gear drum 80 so that the ring 84 trips the switch 142 when the ring 84 reaches the proximate end of the helical gear drum 80.

In operation, when an operator of the instrument 10 pulls back the firing trigger 20, the sensor 110 detects the deployment of the firing trigger 20 and sends a signal to the motor 65 to cause forward rotation of the motor 65 at, for example, a rate proportional to how hard the operator pulls back the firing trigger 20. The forward rotation of the motor 65 in turn causes the ring gear 78 at the distal end of the planetary gear assembly 72 to rotate, thereby causing the helical gear drum 80 to rotate, causing the ring 84 threaded on the helical gear drum 80 to travel distally along the helical gear drum 80. The rotation of the helical gear drum 80 also drives the main drive shaft assembly as described above, which in turn causes deployment of the knife 32 in the end effector 12. That is, the knife 32 and sled 33 are caused to traverse the channel 22 longitudinally, thereby cutting tissue clamped in the end effector 12. Also, the stapling operation of the end effector 12 is caused to happen in embodiments where a stapling-type end effector is used.

By the time the cutting/stapling operation of the end effector 12 is complete, the ring 84 on the helical gear drum 80 will have reached the distal end of the helical gear drum 80, thereby causing the reverse motor sensor 130 to be tripped, which sends a signal to the motor 65 to cause the motor 65 to reverse its rotation. This in turn causes the knife 32 to retract, and also causes the ring 84 on the helical gear drum 80 to move back to the proximate end of the helical gear drum 80.

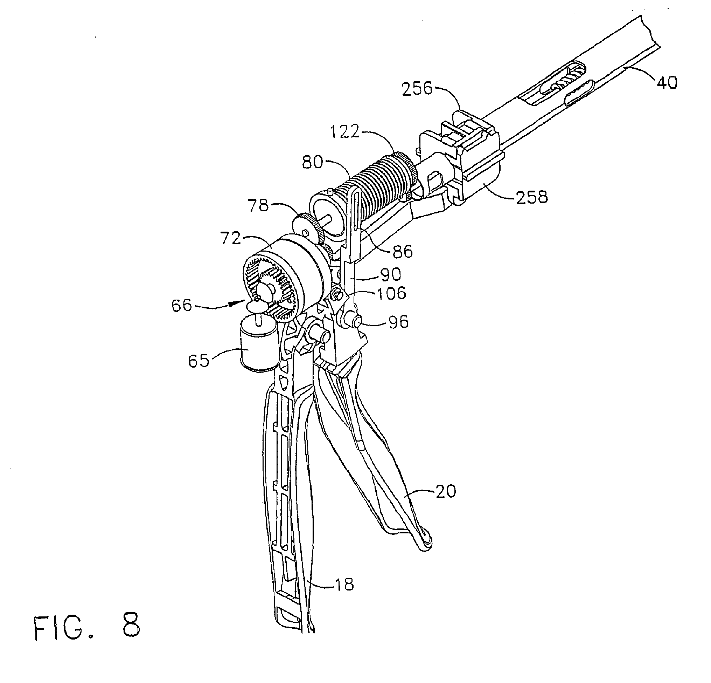

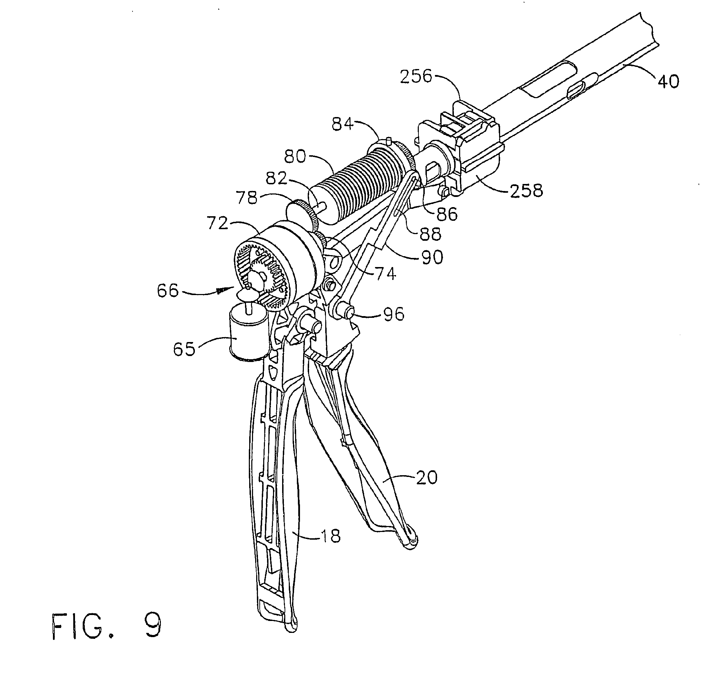

The middle handle piece 104 includes a backside shoulder 106 that engages the slotted arm 90 as best shown in Figures 8 and 9. The middle handle piece 104 also has a forward motion stop 107 that engages the firing trigger 20. The movement of the slotted arm 90 is controlled, as explained above, by rotation of the motor 65. When the slotted arm 90 rotates CCW as the ring 84 travels from the proximate end of the helical gear drum 80 to the distal end, the middle handle piece 104 will be free to rotate CCW. Thus, as the user draws in the firing trigger 20, the firing trigger 20 will engage the forward motion stop 107 of the middle handle piece 104, causing the middle handle piece 104 to rotate CCW. Due to the backside shoulder 106 engaging the slotted arm 90, however, the middle handle piece 104 will only be able to rotate CCW as far as the slotted arm 90 permits. In that way, if the motor 65 should stop rotating for some reason, the slotted arm 90 will stop rotating, and the user will not be able to further draw in the firing trigger 20 because the middle handle piece 104 will not be free to rotate CCW due to the slotted arm 90.

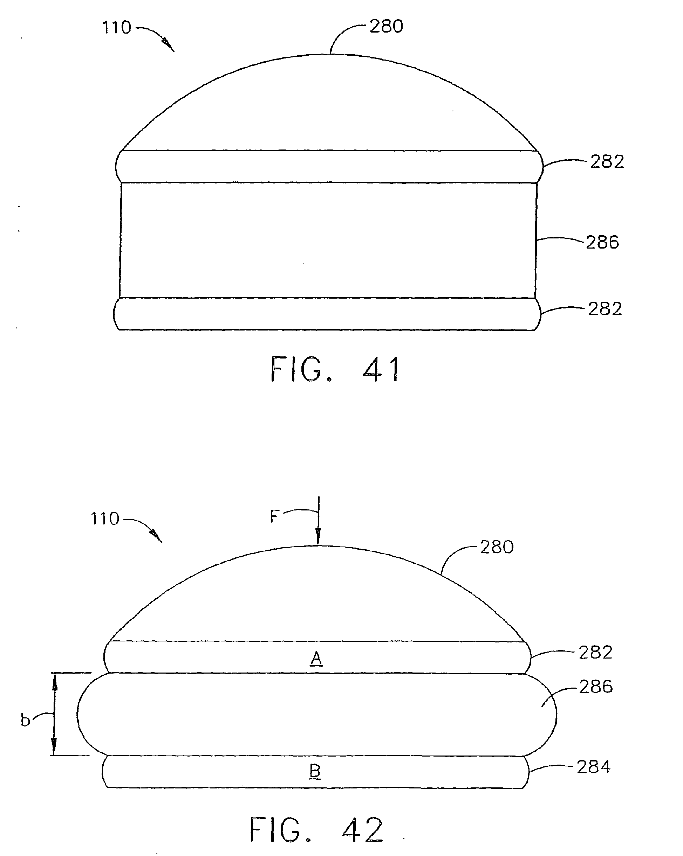

Figure 41 and 42 illustrate two states of a variable sensor that may be used as the run motor sensor 110 according to various embodiments of the present invention. The sensor 110 may include a face portion 280, a first electrode (A) 282, a second electrode (B) 284, and a compressible dielectric material 286 (e.g., EAP) between the electrodes 282, 284. The sensor 110 may be positioned such that the face portion 280 contacts the firing trigger 20 when retracted. Accordingly, when the firing trigger 20 is retracted, the dielectric material 286 is compressed, as shown in Figure 42, such that the electrodes 282, 284 are closer together. Since the distance "b" between the electrodes 282, 284 is directly related to the impedance between the electrodes 282, 284, the greater the distance the more impedance, and the closer the distance the less impedance. In that way, the amount that the dielectric material 286 is compressed due to retraction of the firing trigger 20 (denoted as force "F" in Figure 42) is proportional to the impedance between the electrodes 282, 284, which can be used to proportionally control the motor 65.

Components of an exemplary closure system for closing (or clamping) the anvil 24 of the end effector 12 by retracting the closure trigger 18 are also shown in Figures 7-10. In the illustrated embodiment, the closure system includes a yoke 250 connected to the closure trigger 18 by a pin 251 inserted through aligned openings in both the closure trigger 18 and the yoke 250. A pivot pin 252, about which the closure trigger 18 pivots, is inserted through another opening in the closure trigger 18 which is offset from where the pin 251 is inserted through the closure trigger 18. Thus, retraction of the closure trigger 18 causes the upper part of the closure trigger 18, to which the yoke 250 is attached via the pin 251, to rotate CCW. The distal end of the yoke 250 is connected, via a pin 254, to a first closure bracket 256. The first closure bracket 256 connects to a second closure bracket 258. Collectively, the closure brackets 256, 258 define an opening in which the proximate end of the proximate closure tube 40 (see Figure 4) is seated and held such that longitudinal movement of the closure brackets 256, 258 causes longitudinal motion by the proximate closure tube 40. The instrument 10 also includes a closure rod 260 disposed inside the proximate closure tube 40. The closure rod 260 may include a window 261 into which a post 263 on one of the handle exterior pieces, such as exterior lower side piece 59 in the illustrated embodiment, is disposed to fixedly connect the closure rod 260 to the handle 6. In that way, the proximate closure tube 40 is capable of moving longitudinally relative to the closure rod 260. The closure rod 260 may also include a distal collar 267 that fits into a cavity 269 in proximate spine tube 46 and is retained therein by a cap 271 (see Figure 4).

In operation, when the yoke 250 rotates due to retraction of the closure trigger 18, the closure brackets 256, 258 cause the proximate closure tube 40 to move distally (i.e., away from the handle end of the instrument 10), which causes the distal closure tube 42 to move distally, which causes the anvil 24 to rotate about the pivot pins 25 into the clamped or closed position. When the closure trigger 18 is unlocked from the locked position, the proximate closure tube 40 is caused to slide proximally, which causes the distal closure tube 42 to slide proximally, which, by virtue of the tab 27 being inserted in the opening 45 of the distal closure tube 42, causes the anvil 24 to pivot about the pivot pins 25 into the open or unclamped position. In that way, by retracting and locking the closure trigger 18, an operator may clamp tissue between the anvil 24 and channel 22, and may unclamp the tissue following the cutting/stapling operation by unlocking the closure trigger 18 from the locked position.

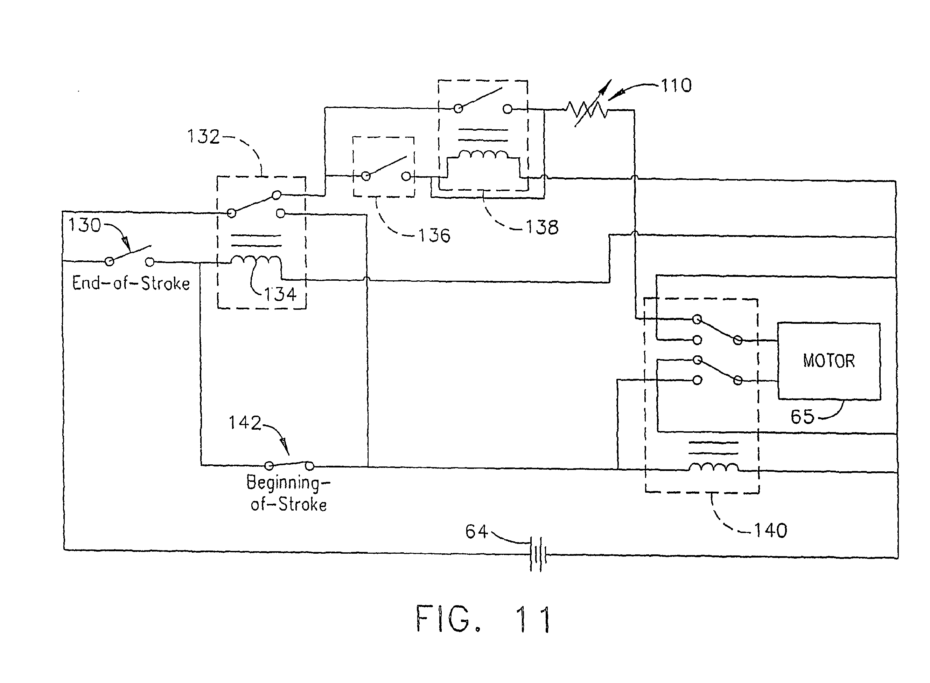

Figure 11 is a schematic diagram of an electrical circuit of the instrument 10 according to various embodiments of the present invention. When an operator initially pulls in the firing trigger 20 after locking the closure trigger 18, the sensor 110 is activated, allowing current to flow therethrough. If the normally-open reverse motor sensor switch 130 is open (meaning the end of the end effector stroke has not been reached), current will flow to a single pole, double throw relay 132. Since the reverse motor sensor switch 130 is not closed, coil 134 of the relay 132 will not be energized, so the relay 132 will be in its non-energized state. The circuit also includes a cartridge lockout sensor 136. If the end effector 12 includes a staple cartridge 34, the sensor 136 will be in the closed state, allowing current to flow. Otherwise, if the end effector 12 does not include a staple cartridge 34, the sensor 136 will be open, thereby preventing the battery 64 from powering the motor 65.

When the staple cartridge 34 is present, the sensor 136 is closed, which energizes a single pole, single throw relay 138. When the relay 138 is energized, current flows through the relay 138, through the variable resistor sensor 110, and to the motor 65 via a double pole, double throw relay 140, thereby powering the motor 65 and allowing it to rotate in the forward direction.

When the end effector 12 reaches the end of its stroke, the reverse motor sensor 130 will be activated, thereby closing the switch 130 and energizing the relay 132. This causes the relay 132 to assume its energized state (not shown in Figure 11), which causes current to bypass the cartridge lockout sensor 136 and variable resistor 110, and instead causes current to flow to both the normally-closed double pole, double throw relay 140 and back to the motor 65, but in a manner, via the relay 140, that causes the motor 65 to reverse its rotational direction.

Because the stop motor sensor switch 142 is normally-closed, current will flow back to the relay 132 to keep it closed until the switch 142 opens. When the knife 32 is fully retracted, the stop motor sensor switch 142 is activated, causing the switch 142 to open, thereby removing power from the motor 65.

In other embodiments, rather than a proportional-type sensor 110, an on-off type sensor could be used. In such embodiments, the rate of rotation of the motor 65 would not be proportional to the force applied by the operator. Rather, the motor 65 would generally rotate at a constant rate. But the operator would still experience force feedback because the firing trigger 20 is geared into the gear drive train.

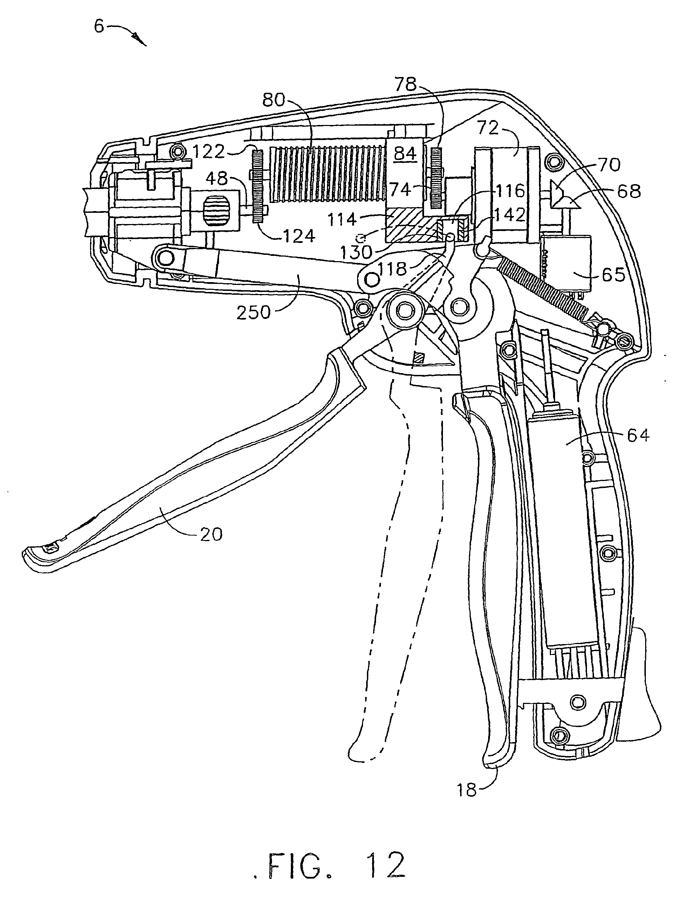

Figure 12 is a side-view of the handle 6 of a power-assist motorized endocutter according to another embodiment. The embodiment of Figure 12 is similar to that of Figures 7-10 except that in the embodiment of Figure 12, there is no slotted arm 90 connected to the ring 84 threaded on the helical gear drum 80. Instead, in the embodiment of Figure 12, the ring 84 includes a sensor portion 114 that moves with the ring 84 as the ring 84 advances down (and back) on the helical gear drum 80. The sensor portion 114 includes a notch 116. The reverse motor sensor 130 may be located at the distal end of the notch 116 and the stop motor sensor 142 may be located at the proximate end of the notch 116. As the ring 84 moves down the helical gear drum 80 (and back), the sensor portion 114 moves with it. Further, as shown in Figure 12, the middle piece 104 may have an arm 118 that extends into the notch 116.

In operation, as an operator of the instrument 10 retracts in the firing trigger 20 toward the pistol grip 26, the run motor sensor 110 detects the motion and sends a signal to power the motor 65, which causes, among other things, the helical gear drum 80 to rotate. As the helical gear drum 80 rotates, the ring 84 threaded on the helical gear drum 80 advances (or retracts, depending on the rotation). Also, due to the pulling in of the firing trigger 20, the middle piece 104 is caused to rotate CCW with the firing trigger 20 due to the forward motion stop 107 that engages the firing trigger 20. The CCW rotation of the middle piece 104 cause the arm 118 to rotate CCW with the sensor portion 114 of the ring 84 such that the arm 118 stays disposed in the notch 116. When the ring 84 reaches the distal end of the helical gear drum 80, the arm 118 will contact and thereby trip the reverse motor sensor 130. Similarly, when the ring 84 reaches the proximate end of the helical gear drum 80, the arm 118 will contact and thereby trip the stop motor sensor 142. Such actions may reverse and stop the motor 65, respectively, as described above.

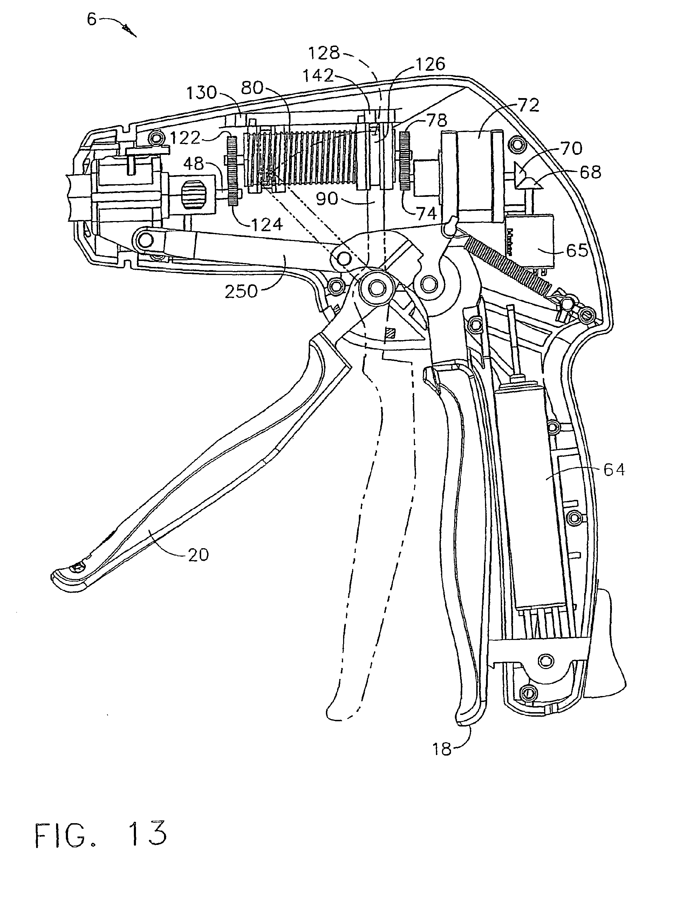

Figure 13 is a side-view of the handle 6 of a power-assist motorized endocutter according to another embodiment. The embodiment of Figure 13 is similar to that of Figures 7-10 except that in the embodiment of Figure 13, there is no slot in the arm 90. Instead, the ring 84 threaded on the helical gear drum 80 includes a vertical channel 126. Instead of a slot, the arm 90 includes a post 128 that is disposed in the channel 126. As the helical gear drum 80 rotates, the ring 84 threaded on the helical gear drum 80 advances (or retracts, depending on the rotation). The arm 90 rotates CCW as the ring 84 advances due to the post 128 being disposed in the channel 126, as shown in Figure 13.

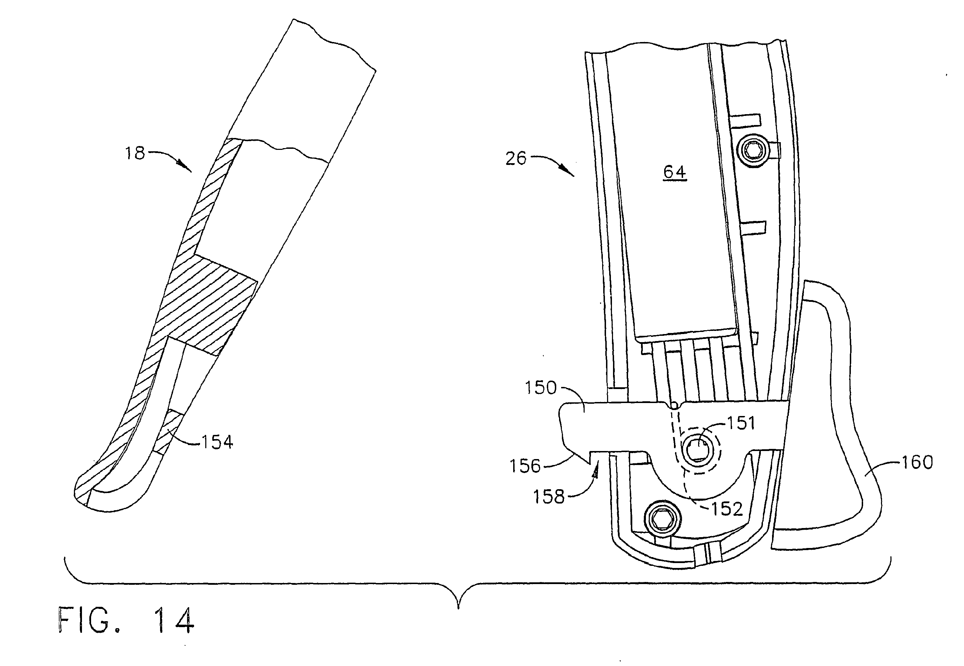

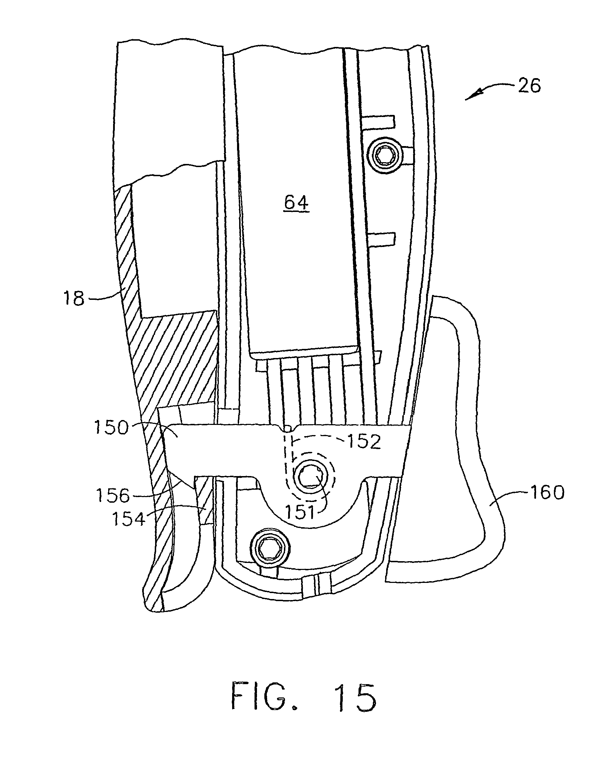

As mentioned above, in using a two-stroke motorized instrument, the operator first pulls back and locks the closure trigger 18. Figures 14 and 15 show one embodiment of a way to lock the closure trigger 18 to the pistol grip portion 26 of the handle 6. In the illustrated embodiment, the pistol grip portion 26 includes a hook 150 that is biased to rotate CCW about a pivot point 151 by a torsion spring 152. Also, the closure trigger 18 includes a closure bar 154. As the operator draws in the closure trigger 18, the closure bar 154 engages a sloped portion 156 of the hook 150, thereby rotating the hook 150 upward (or CW in Figures 14-15) until the closure bar 154 completely passes the sloped portion 156 into a recessed notch 158 of the hook 150, which locks the closure trigger 18 in place. The operator may release the closure trigger 18 by pushing down on a slide button release 160 on the back or opposite side of the pistol grip portion 26. Pushing down the slide button release 160 rotates the hook 150 CW such that the closure bar 154 is released from the recessed notch 158.

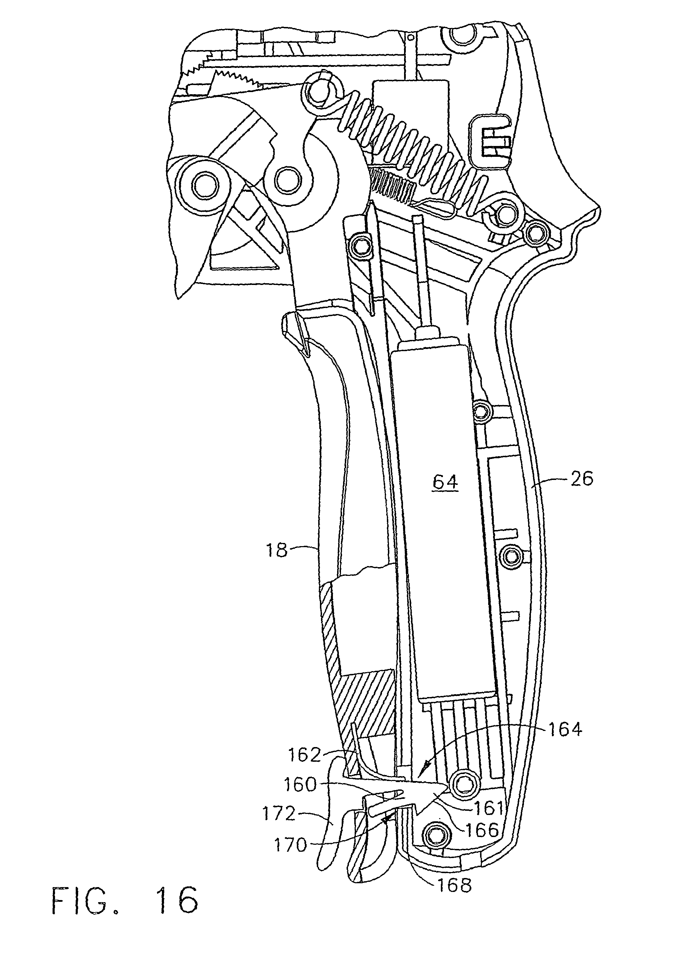

Figure 16 shows another closure trigger locking mechanism according to various embodiments. In the embodiment of Figure 16, the closure trigger 18 includes a wedge 160 having an arrow-head portion 161. The arrow-head portion 161 is biased downward (or CW) by a leaf spring 162. The wedge 160 and leaf spring 162 may be made from, for example, molded plastic. When the closure trigger 18 is retracted, the arrow-head portion 161 is inserted through an opening 164 in the pistol grip portion 26 of the handle 6. A lower chamfered surface 166 of the arrow-head portion 161 engages a lower sidewall 168 of the opening 164, forcing the arrow-head portion 161 to rotate CCW. Eventually the lower chamfered surface 166 fully passes the lower sidewall 168, removing the CCW force on the arrow-head portion 161, causing the lower sidewall 168 to slip into a locked position in a notch 170 behind the arrow-head portion 161.

To unlock the closure trigger 18, a user presses down on a button 172 on the opposite side of the closure trigger 18, causing the arrow-head portion 161 to rotate CCW and allowing the arrow-head portion 161 to slide out of the opening 164.

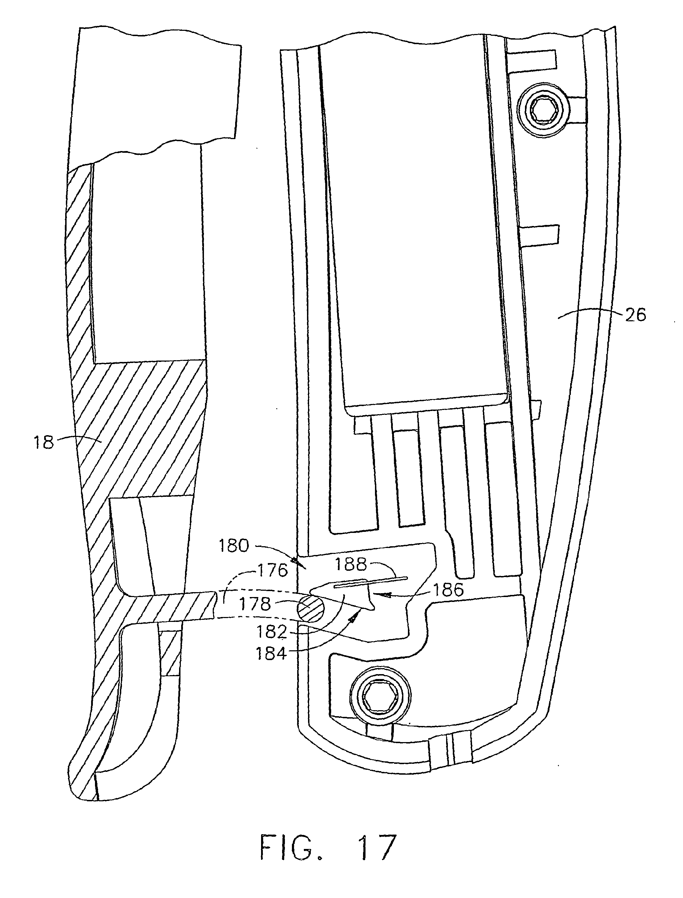

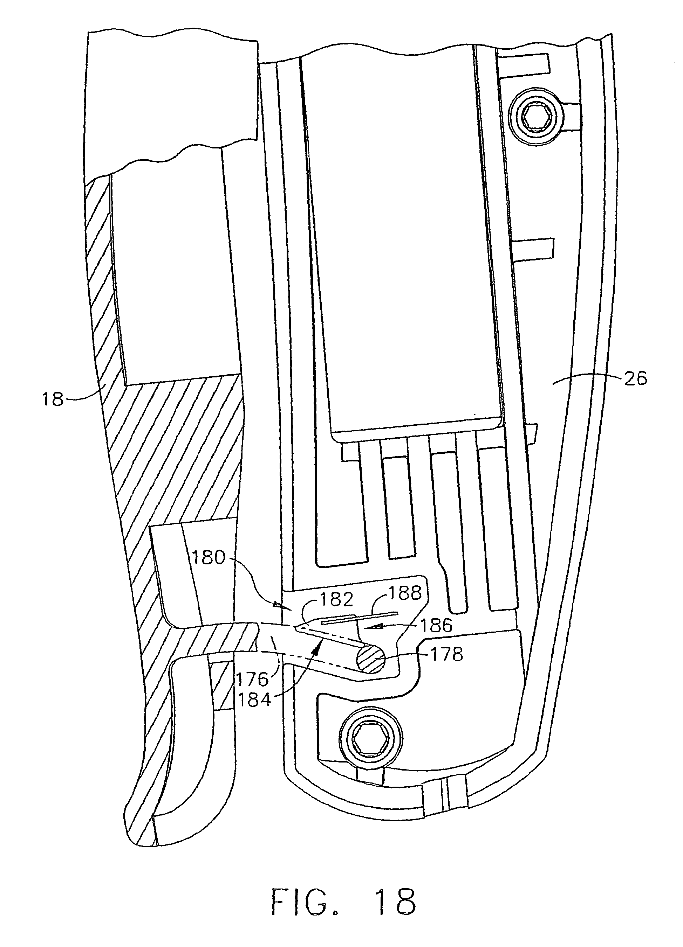

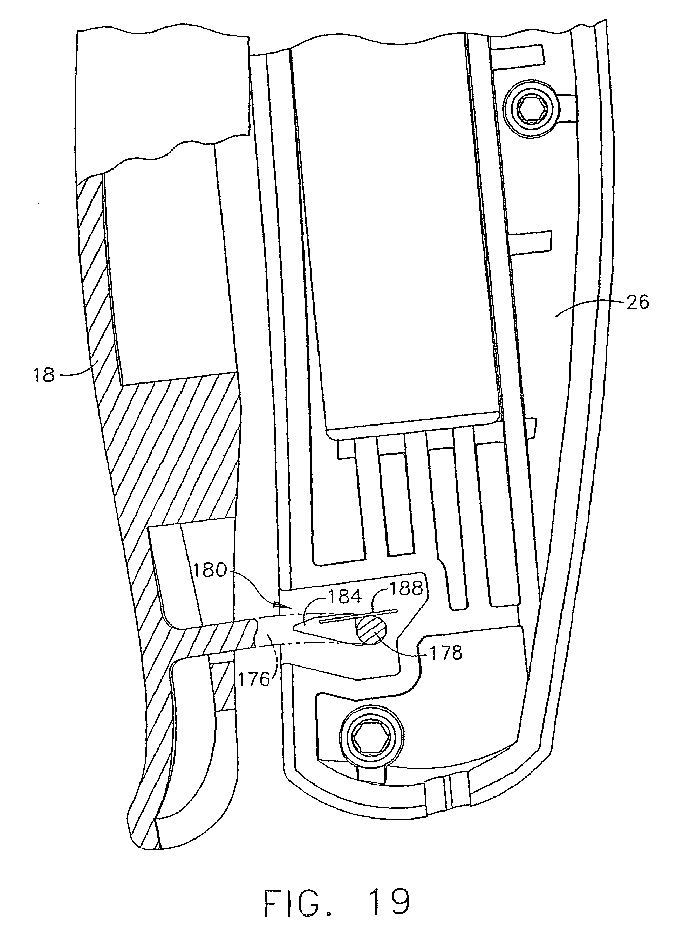

Figures 17-22 show a closure trigger locking mechanism according to another embodiment. As shown in this embodiment, the closure trigger 18 includes a flexible longitudinal arm 176 that includes a lateral pin 178 extending therefrom. The arm 176 and pin 178 may be made from molded plastic, for example. The pistol grip portion 26 of the handle 6 includes an opening 180 with a laterally extending wedge 182 disposed therein. When the closure trigger 18 is retracted, the pin 178 engages the wedge 182, and the pin 178 is forced downward (i.e., the arm 176 is rotated CW) by the lower surface 184 of the wedge 182, as shown in Figures 17 and 18. When the pin 178 fully passes the lower surface 184, the CW force on the arm 176 is removed, and the pin 178 is rotated CCW such that the pin 178 comes to rest in a notch 186 behind the wedge 182, as shown in Figure 19, thereby locking the closure trigger 18. The pin 178 is further held in place in the locked position by a flexible stop 188 extending from the wedge 184.

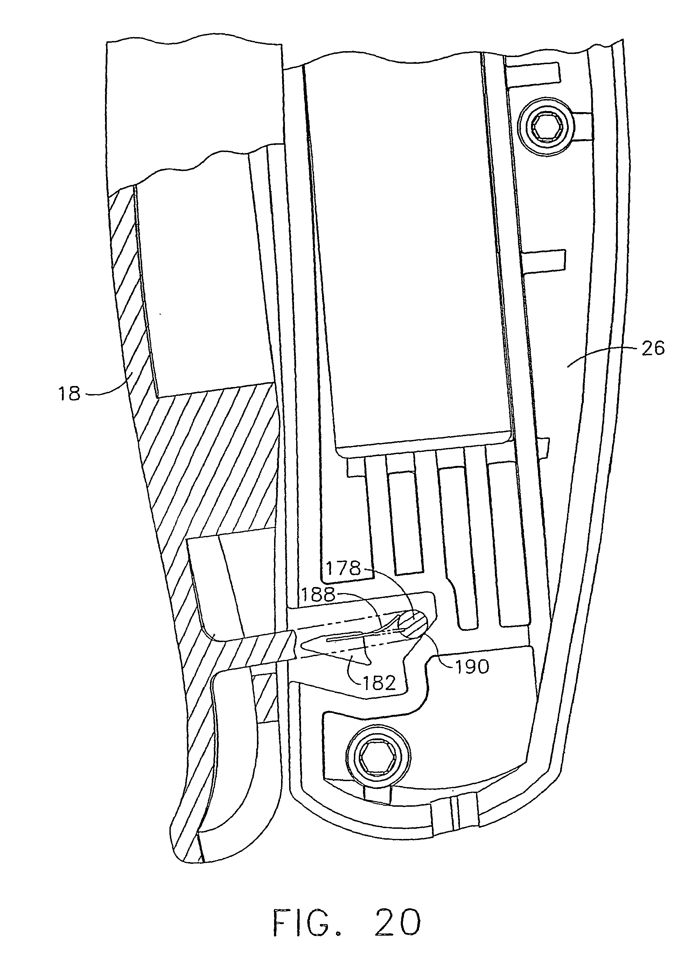

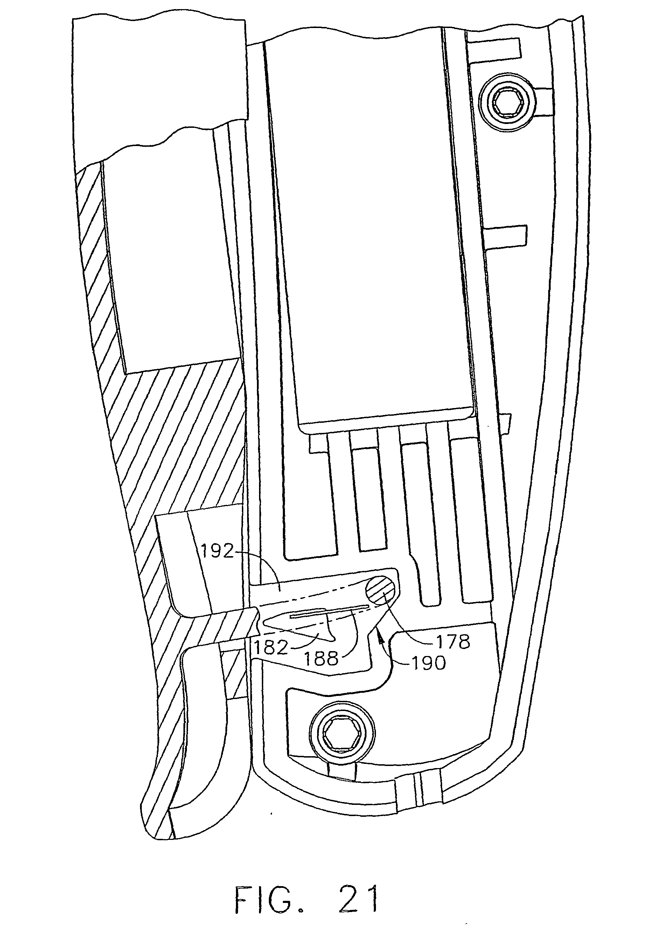

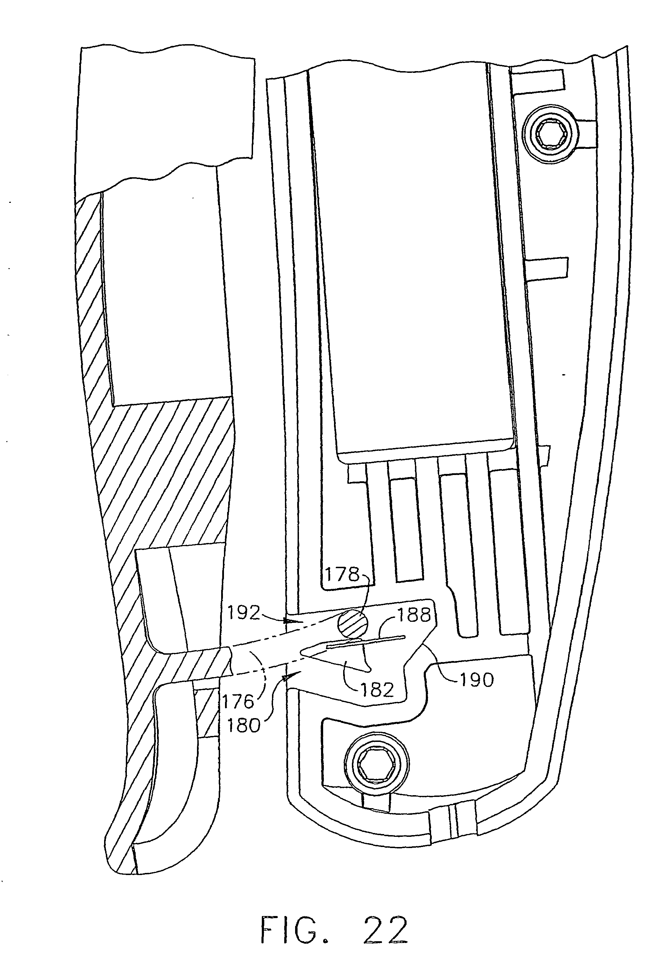

To unlock the closure trigger 18, the operator may further squeeze the closure trigger 18, causing the pin 178 to engage a sloped backwall 190 of the opening 180, forcing the pin 178 upward past the flexible stop 188, as shown in Figures 20 and 21. The pin 178 is then free to travel out an upper channel 192 in the opening 180 such that the closure trigger 18 is no longer locked to the pistol grip portion 26, as shown in Figure 22.

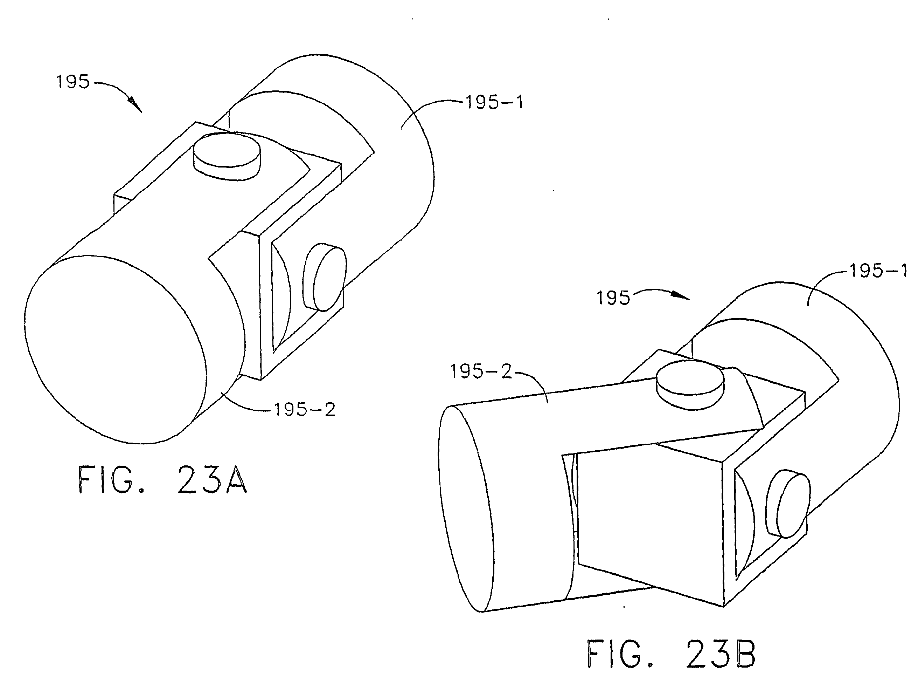

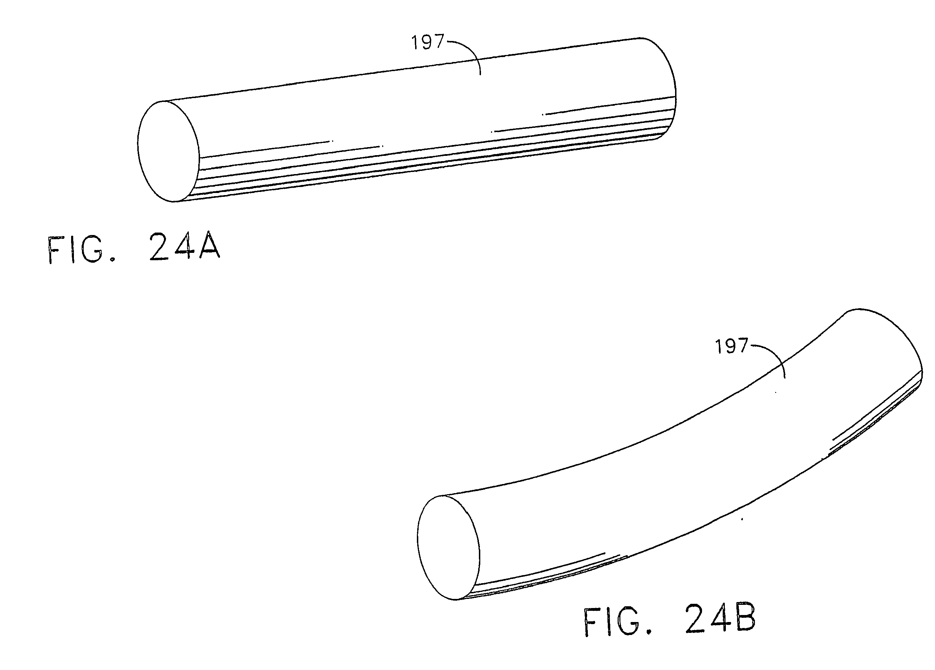

Figures 23A-B show a universal joint ("u-joint") 195. The second piece 195-2 of the u-joint 195 rotates in a horizontal plane in which the first piece 195-1 lies. Figure 23A shows the u-joint 195 in a linear (180°) orientation and Figure 23B shows the u-joint 195 at approximately a 150° orientation. The u-joint 195 may be used instead of the bevel gears 52a-c (see Figure 4, for example) at the articulation point 14 of the main drive shaft assembly to articulate the end effector 12. Figures 24A-B show a torsion cable 197 that may be used in lieu of both the bevel gears 52a-c and the u-joint 195 to realize articulation of the end effector 12.

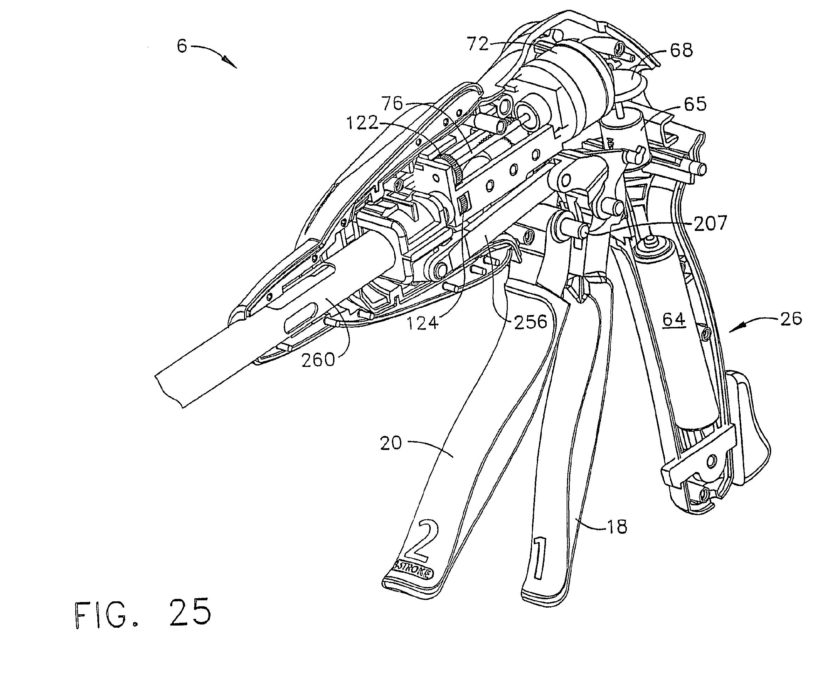

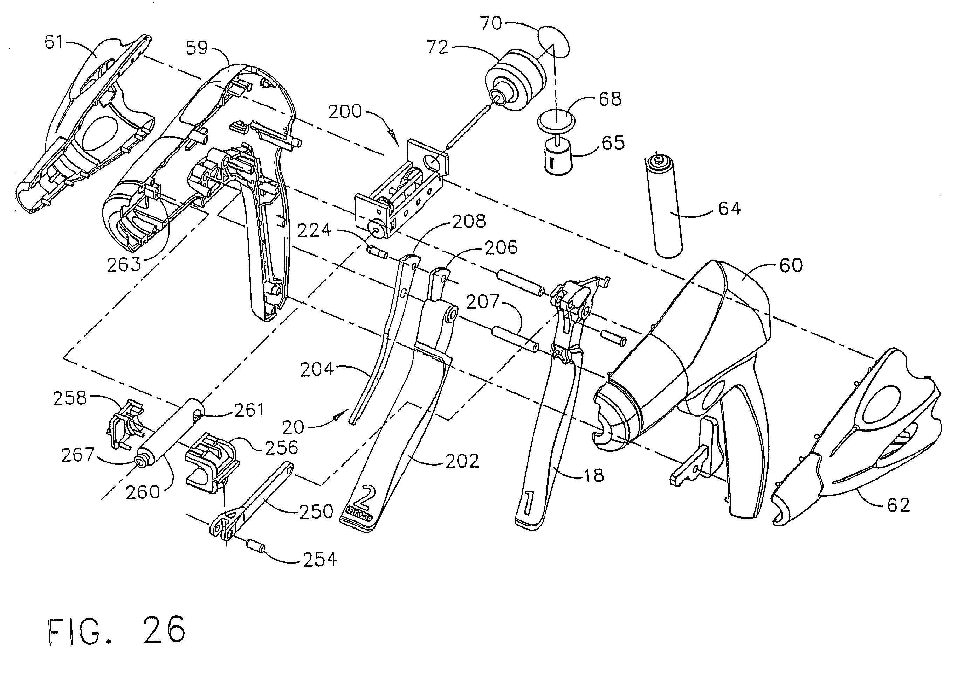

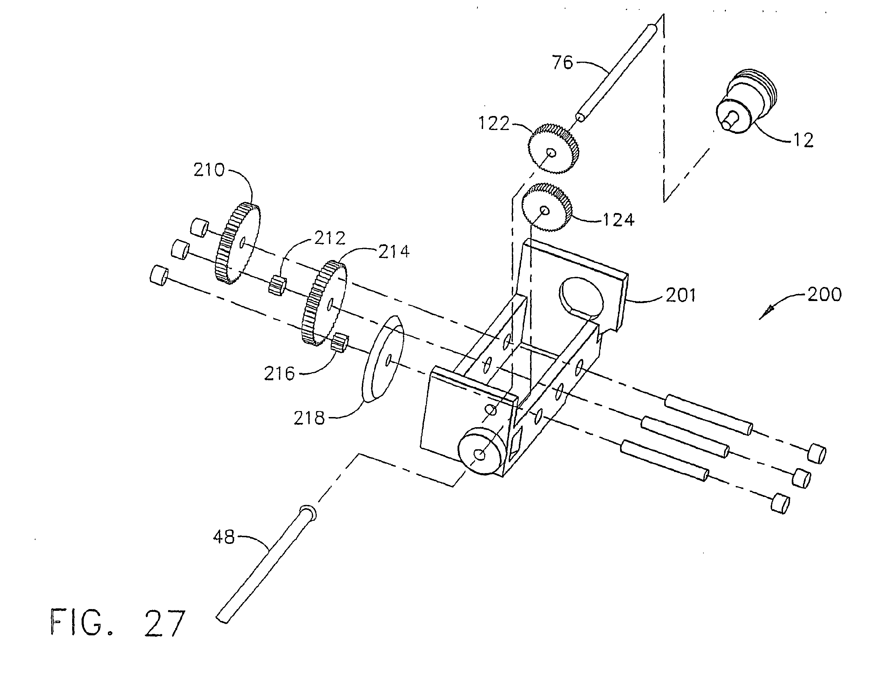

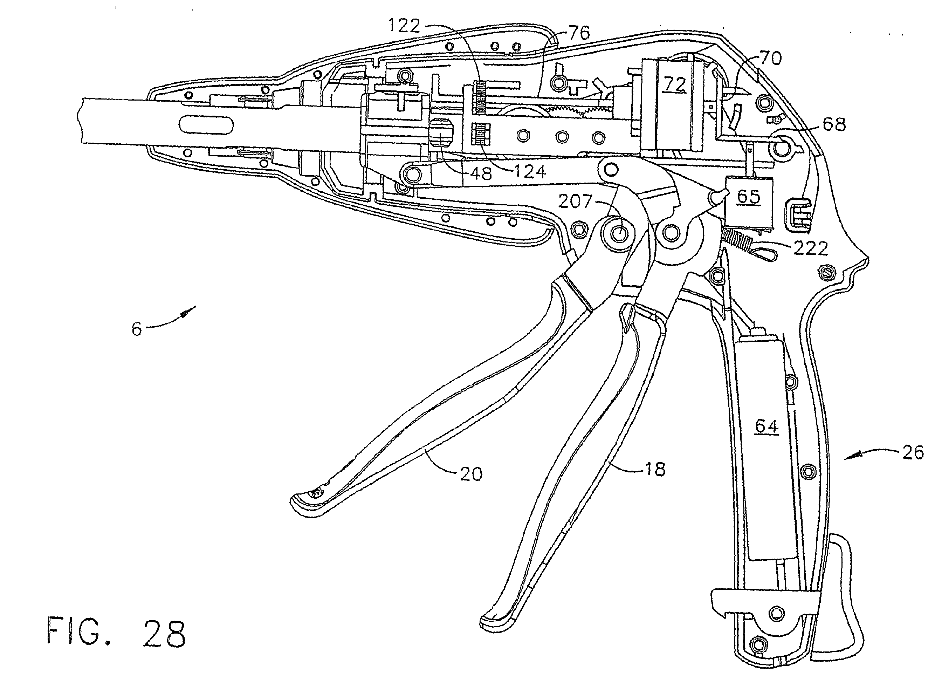

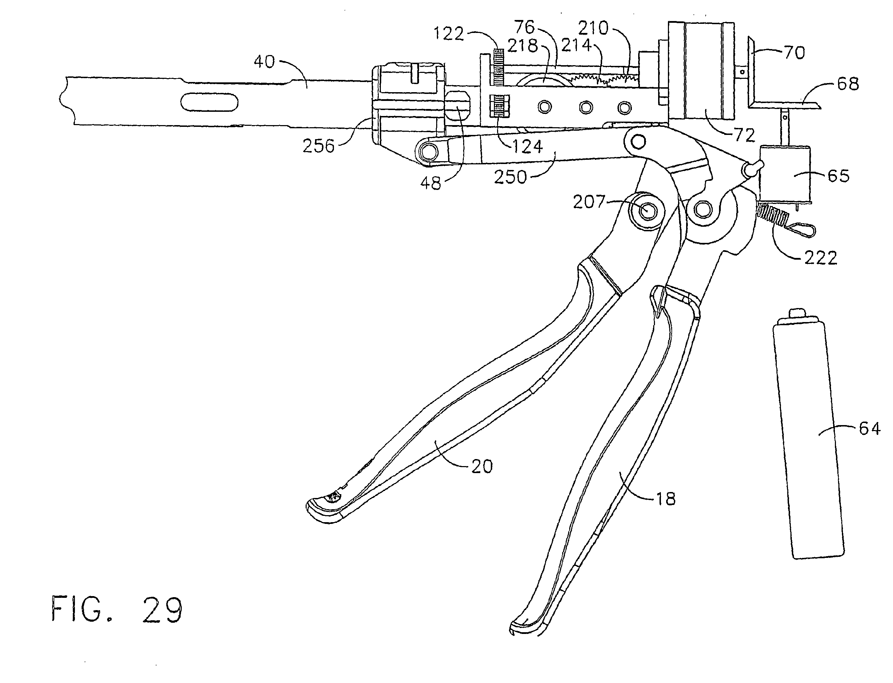

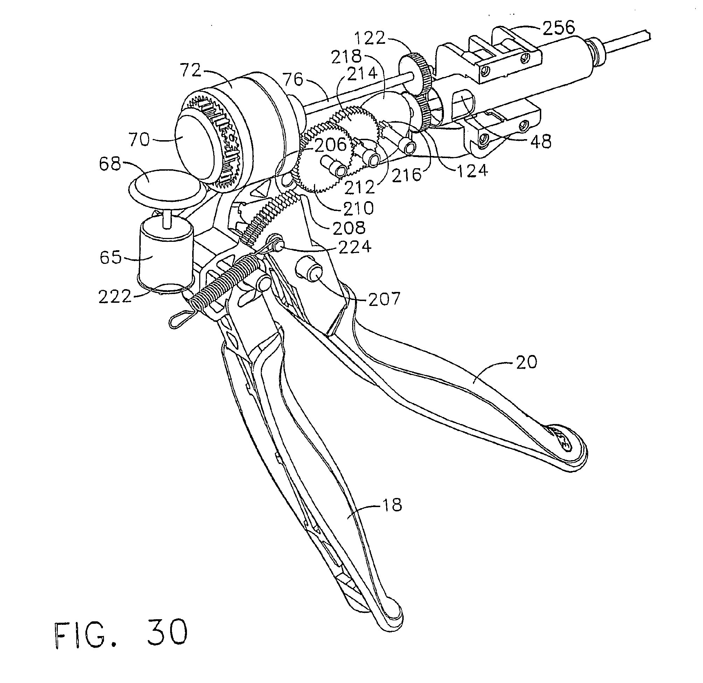

Figures 25-31 illustrate another embodiment of a motorized, two-stroke endoscopic surgical instrument 10 with power assist according to another embodiment of the present invention. The embodiment of Figures 25-31 is similar to that of Figures 6-10 except that instead of the helical gear drum 80, the embodiment of Figures 25-31 includes an alternative gear drive assembly. The embodiment of Figures 25-31 includes a gear box assembly 200 including a number of gears disposed in a frame 201, wherein the gears are connected between the planetary gear 72 and the pinion gear 124 at the proximate end of the drive shaft 48. As explained further below, the gear box assembly 200 provides feedback to the user via the firing trigger 20 regarding the deployment and loading force of the end effector 12. Also, the user may provide power to the system via the gear box assembly 200 to assist the deployment of the end effector 12. In that sense, like the embodiments described above, the embodiment of Figures 25-31 is another power assist, motorized instrument 10 that provides feedback to the user regarding the loading force experienced by the cutting instrument 32.

In the illustrated embodiment, the firing trigger 20 includes two pieces: a main body portion 202 and a stiffening portion 204. The main body portion 202 may be made of plastic, for example, and the stiffening portion 204 may be made out of a more rigid material, such as metal. In the illustrated embodiment, the stiffening portion 204 is adjacent to the main body portion 202, but according to other embodiments, the stiffening portion 204 could be disposed inside the main body portion 202. A pivot pin 207 may be inserted through openings in the firing trigger pieces 202, 204 and may be the point about which the firing trigger 20 rotates. In addition, a spring 222 may bias the firing trigger 20 to rotate in a CCW direction. The spring 222 may have a distal end connected to a pin 224 that is connected to the pieces 202, 204 of the firing trigger 20. The proximate end of the spring 222 may be connected to one of the handle exterior lower side pieces 59, 60.

In the illustrated embodiment, both the main body portion 202 and the stiffening portion 204 include gear portions 206, 208 (respectively) at their upper end portions. The gear portions 206, 208 engage a gear in the gear box assembly 200, as explained below, to drive the main drive shaft assembly and to provide feedback to the user regarding the deployment of the end effector 12.

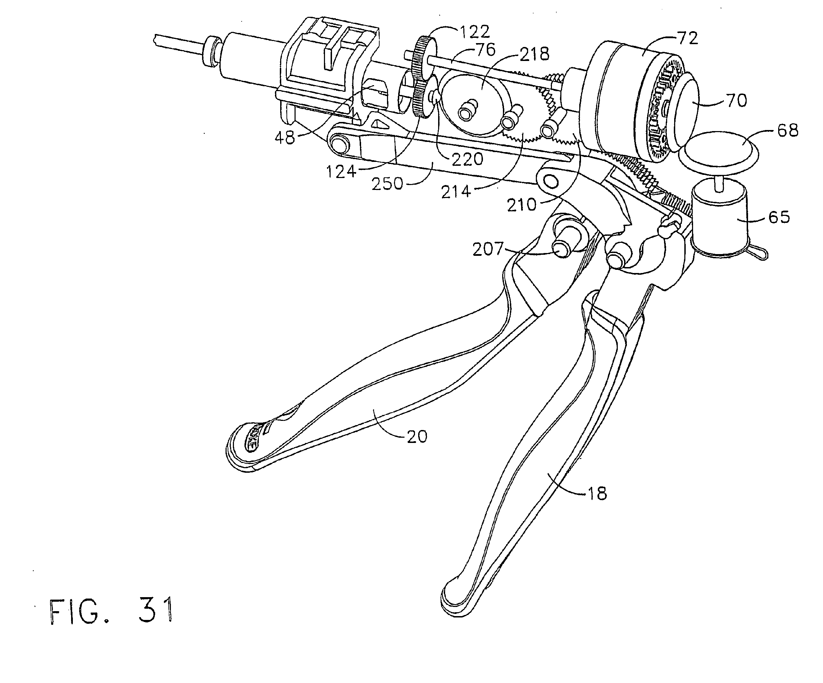

The gear box assembly 200 may include as shown, in the illustrated embodiment, six (6) gears. A first gear 210 of the gear box assembly 200 engages the gear portions 206, 208 of the firing trigger 20. In addition, the first gear 210 engages a smaller second gear 212, the smaller second gear 212 being coaxial with a large third gear 214. The third gear 214 engages a smaller fourth gear 216, the smaller fourth gear 216 being coaxial with a fifth gear 218. The fifth gear 218 is a 90° bevel gear that engages a mating 90° bevel gear 220 (best shown in Fig. 31) that is connected to the pinion gear 124 that drives the main drive shaft 48.

In operation, when the user retracts the firing trigger 20, a run motor sensor (not shown) is activated, which may provide a signal to the motor 65 to rotate at a rate proportional to the extent or force with which the operator is retracting the firing trigger 20. This causes the motor 65 to rotate at a speed proportional to the signal from the sensor. The sensor is not shown for this embodiment, but it could be similar to the run motor sensor 110 described above. The sensor could be located in the handle 6 such that it is depressed when the firing trigger 20 is retracted. Also, instead of a proportional-type sensor, an on/off type sensor may be used.

Rotation of the motor 65 causes the bevel gears 66, 70 to rotate, which causes the planetary gear 72 to rotate, which causes, via the drive shaft 76, the ring gear 122 to rotate. The ring gear 122 meshes with the pinion gear 124, which is connected to the main drive shaft 48. Thus, rotation of the pinion gear 124 drives the main drive shaft 48, which causes actuation of the cutting/stapling operation of the end effector 12.

Forward rotation of the pinion gear 124 in turn causes the bevel gear 220 to rotate, which causes, by way of the rest of the gears of the gear box assembly 200, the first gear 210 to rotate. The first gear 210 engages the gear portions 206, 208 of the firing trigger 20, thereby causing the firing trigger 20 to rotate CCW when the motor 65 provides forward drive for the end effector 12 (and to rotate CCW when the motor 65 rotates in reverse to retract the end effector 12). In that way, the user experiences feedback regarding loading force and deployment of the end effector 12 by way of the user's grip on the firing trigger 20. Thus, when the user retracts the firing trigger 20, the operator will experience a resistance related to the load force experienced by the end effector 12. Similarly, when the operator releases the firing trigger 20 after the cutting/stapling operation so that it can return to its original position, the user will experience a CW rotation force from the firing trigger 20 that is generally proportional to the reverse speed of the motor 65.

It should also be noted that in this embodiment the user can apply force (either in lieu of or in addition to the force from the motor 65) to actuate the main drive shaft assembly (and hence the cutting/stapling operation of the end effector 12) through retracting the firing trigger 20. That is, retracting the firing trigger 20 causes the gear portions 206, 208 to rotate CCW, which causes the gears of the gear box assembly 200 to rotate, thereby causing the pinion gear 124 to rotate, which causes the main drive shaft 48 to rotate.

Although not shown in Figures 25-31, the instrument 10 may further include reverse motor and stop motor sensors. As described above, the reverse motor and stop motor sensors may detect, respectively, the end of the cutting stroke (full deployment of the knife 32 and sled 33) and the end of retraction operation (full retraction of the knife/sled driving member 32). A circuit similar to that described above in connection with Figure 11 may be used to appropriately power the motor 65.

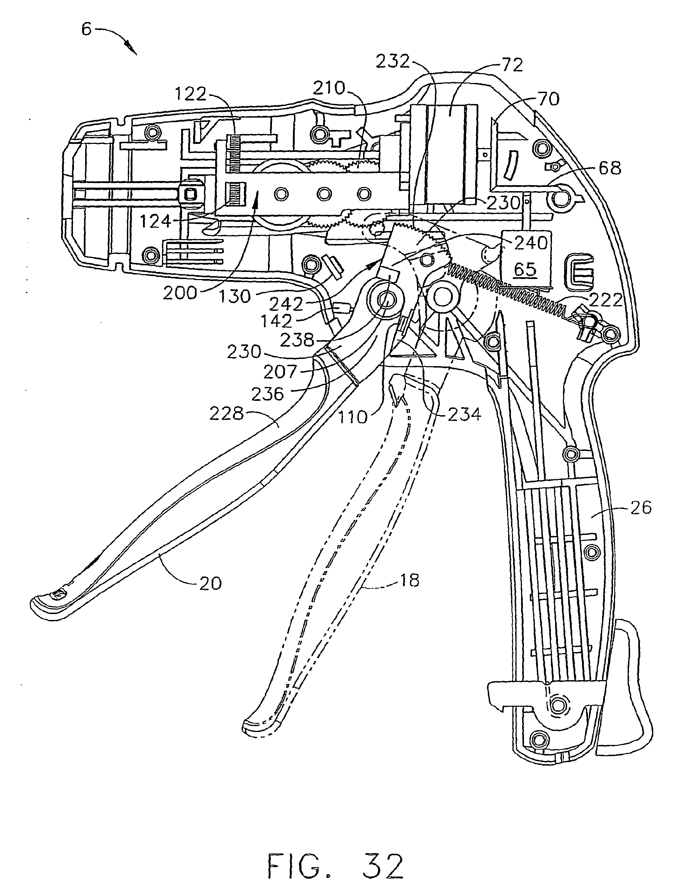

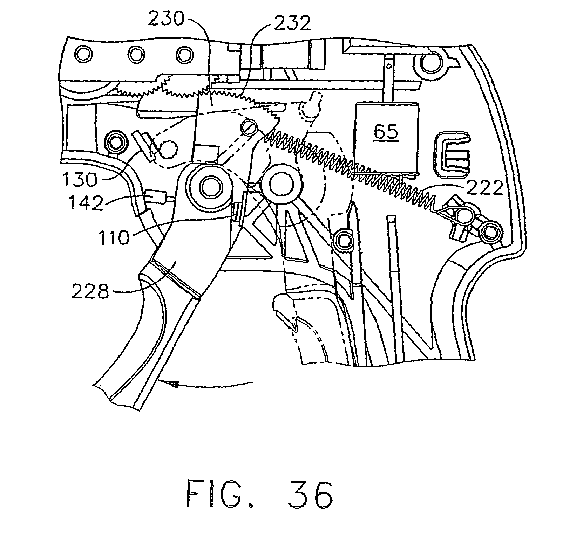

Figures 32-36 illustrate a two-stroke, motorized endoscopic surgical instrument 10 with power assist according to another embodiment. The embodiment of Figures 32-36 is similar to that of Figures 25-31 except that in the embodiment of Figures 32-36, the firing trigger 20 includes a lower portion 228 and an upper portion 230. Both portions 228, 230 are connected to and pivot about a pivot pin 207 that is disposed through each portion 228, 230. The upper portion 230 includes a gear portion 232 that engages the first gear 210 of the gear box assembly 200. The spring 222 is connected to the upper portion 230 such that the upper portion is biased to rotate in the CW direction. The upper portion 230 may also include a lower arm 234 that contacts an upper surface of the lower portion 228 of the firing trigger 20 such that when the upper portion 230 is caused to rotate CW the lower portion 228 also rotates CW, and when the lower portion 228 rotates CCW the upper portion 230 also rotates CCW. Similarly, the lower portion 228 includes a rotational stop 238 that engages a lower shoulder of the upper portion 230. In that way, when the upper portion 230 is caused to rotate CCW the lower portion 228 also rotates CCW, and when the lower portion 228 rotates CW the upper portion 230 also rotates CW.

The illustrated embodiment also includes the run motor sensor 110 that communicates a signal to the motor 65 that, in various embodiments, may cause the motor 65 to rotate at a speed proportional to the force applied by the operator when retracting the firing trigger 20. The sensor 110 may be, for example, a rheostat or some other variable resistance sensor, as explained herein. In addition, the instrument 10 may include a reverse motor sensor 130 that is tripped or switched when contacted by a front face 242 of the upper portion 230 of the firing trigger 20. When activated, the reverse motor sensor 130 sends a signal to the motor 65 to reverse direction. Also, the instrument 10 may include a stop motor sensor 142 that is tripped or actuated when contacted by the lower portion 228 of the firing trigger 20. When activated, the stop motor sensor 142 sends a signal to stop the reverse rotation of the motor 65.

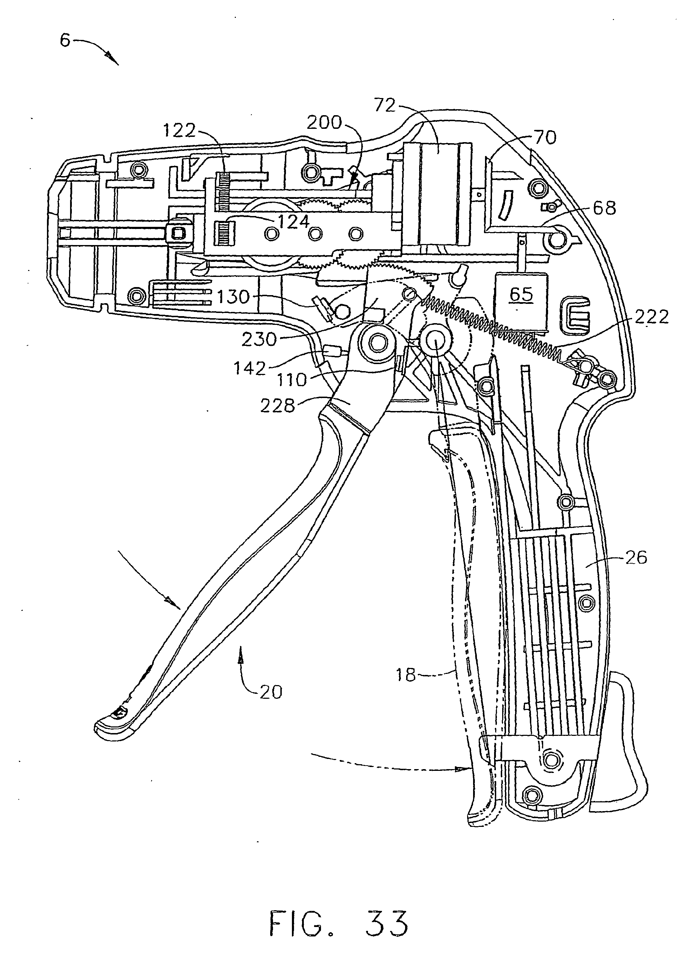

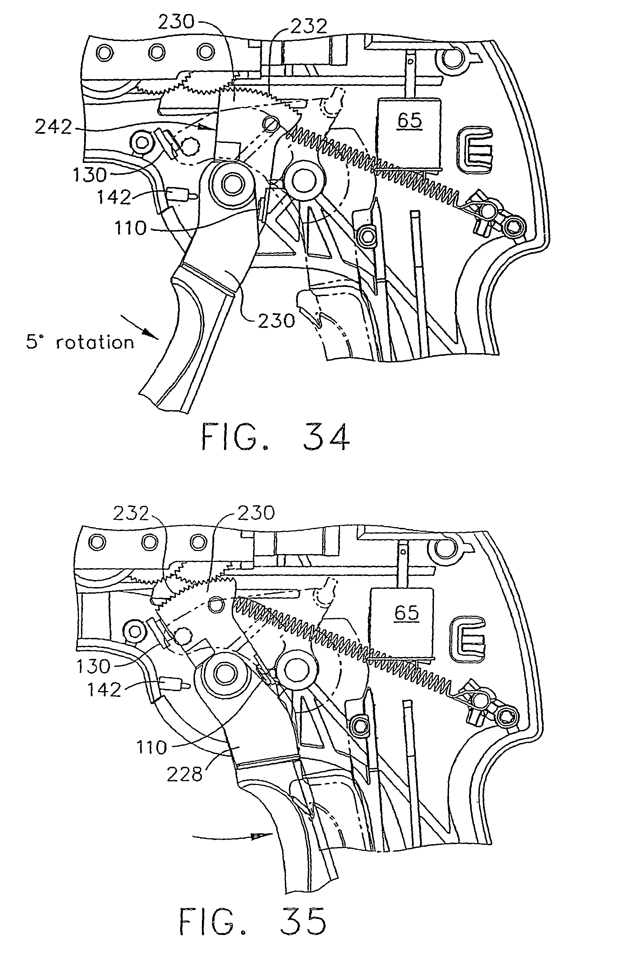

In operation, when an operator retracts the closure trigger 18 into the locked position, the firing trigger 20 is retracted slightly (through mechanisms known in the art, including U.S. Pat. No. 6,905,057 entitled "Surgical Stapling Instrument incorporating a Firing Mechanism having a Linked Rack Transmission" to Swayze et al., which is incorporated herein by reference) so that the user can grasp the firing trigger 20 to initiate the cutting/stapling operation, as shown in Figures 32 and 33. At that point, as shown in Figure 33, the gear portion 232 of the upper portion 230 of the firing trigger 20 moves into engagement with the first gear 210 of the gear box assembly 200. When the operator retracts the firing trigger 20, according to various embodiments, the firing trigger 20 may rotate a small amount, such as five degrees, before tripping the run motor sensor 110, as shown in Figure 34. Activation of the sensor 110 causes the motor 65 to forward rotate at a rate proportional to the retraction force applied by the operator. The forward rotation of the motor 65 causes, as described above, the main drive shaft 48 to rotate, which causes the knife 32 in the end effector 12 to be deployed (i.e., begin traversing the channel 22). Rotation of the pinion gear 124, which is connected to the main drive shaft 48, causes the gears 210-220 in the gear box assembly 200 to rotate. Since the first gear 210 is in engagement with the gear portion 232 of the upper portion 230 of the firing trigger 20, the upper portion 230 is caused to rotate CCW, which causes the lower portion 228 to also rotate CCW.

When the knife 32 is fully deployed (i.e., at the end of the cutting stroke), the front face 242 of the upper portion 230 trips the reverse motor sensor 130, which sends a signal to the motor 65 to reverse rotational direction. This causes the main drive shaft assembly to reverse rotational direction to retract the knife 32. Reverse rotation of the main drive shaft assembly causes the gears 210-220 in the gear box assembly 200 to reverse direction, which causes the upper portion 230 of the firing trigger 20 to rotate CW, which causes the lower portion 228 of the firing trigger 20 to rotate CW until the front face 242 of the upper portion 230 trips or actuates the stop motor sensor 142 when the knife 32 is fully retracted, which causes the motor 65 to stop. In that way, the user experiences feedback regarding deployment of the end effector 12 by way of the user's grip on the firing trigger 20. Thus, when the user retracts the firing trigger 20, the operator will experience a resistance related to the deployment of the end effector 12 and, in particular, to the loading force experienced by the knife 32. Similarly, when the operator releases the firing trigger 20 after the cutting/stapling operation so that it can return to its original position, the user will experience a CW rotation force from the firing trigger 20 that is generally proportional to the reverse speed of the motor 65.

It should also be noted that in this embodiment the user can apply force (either in lieu of or in addition to the force from the motor 65) to actuate the main drive shaft assembly (and hence the cutting/stapling operation of the end effector 12) through retracting the firing trigger 20. That is, retracting the firing trigger 20 causes the gear portion 232 of the upper portion 230 to rotate CCW, which causes the gears of the gear box assembly 200 to rotate, thereby causing the pinion gear 124 to rotate, which causes the main drive shaft assembly to rotate.

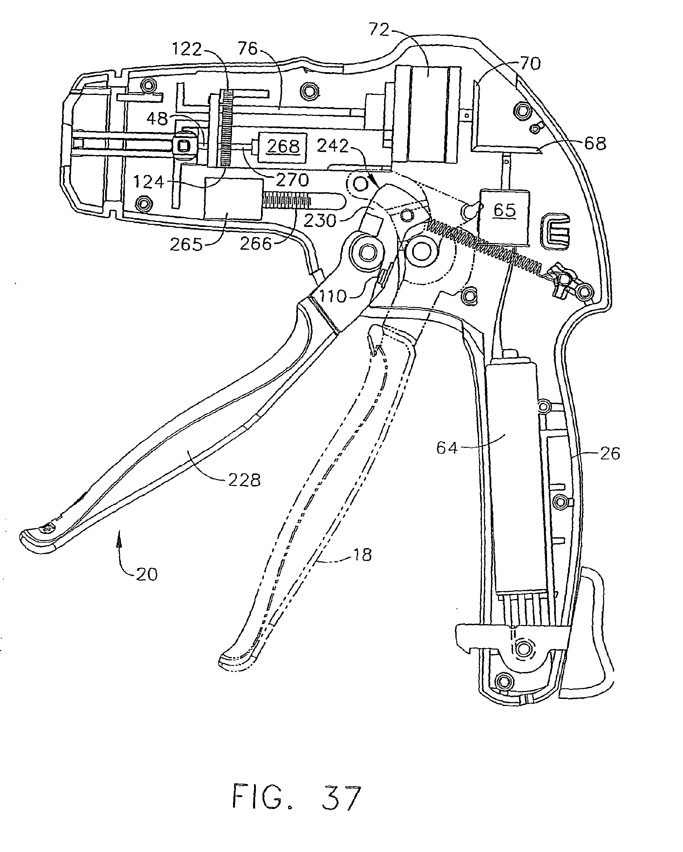

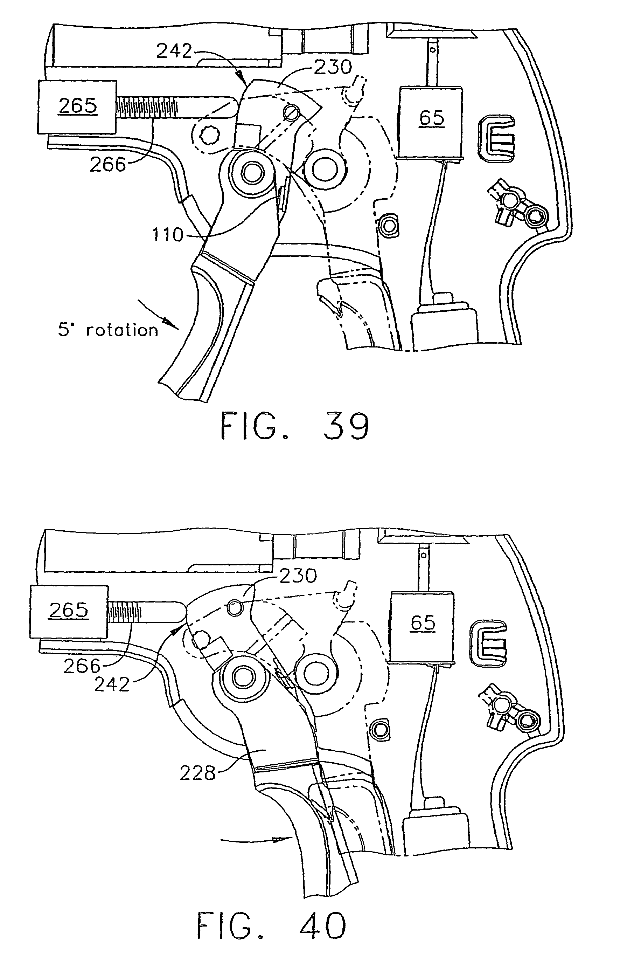

The above-described embodiments employed power-assist user feedback systems, with or without adaptive control (e.g., using a sensor 110, 130, and 142 outside of the closed loop system of the motor, gear drive train, and end effector) for a two-stroke, motorized endoscopic surgical instrument. That is, force applied by the user in retracting the firing trigger 20 may be added to the force applied by the motor 65 by virtue of the firing trigger 20 being geared into (either directly or indirectly) the gear drive train between the motor 65 and the main drive shaft 48. In other embodiments of the present invention, the user may be provided with tactile feedback regarding the position of the knife 32 in the end effector 12, but without having the firing trigger 20 geared into the gear drive train. Figures 37-40 illustrate a motorized endoscopic surgical instrument 10 with such a tactile position feedback system.

In the illustrated embodiment of Figures 37-40, the firing trigger 20 may have a lower portion 228 and an upper portion 230, similar to the instrument 10 shown in Figures 32-36. Unlike the embodiment of Figure 32-36, however, the upper portion 230 does not have a gear portion that mates with part of the gear drive train. Instead, the instrument 10 includes a second motor 265 with a threaded rod 266 threaded therein. The threaded rod 266 reciprocates longitudinally in and out of the motor 265 as the motor 265 rotates, depending on the direction of rotation. The instrument 10 also includes an encoder 268 that is responsive to the rotations of the main drive shaft 48 for translating the incremental angular motion of the main drive shaft 48 (or other component of the main drive assembly) into a corresponding series of digital signals, for example. In the illustrated embodiment, the pinion gear 124 includes a proximate drive shaft 270 that connects to the encoder 268.

The instrument 10 also includes a control circuit (not shown), which may be implemented using a microcontroller or some other type of integrated circuit, that receives the digital signals from the encoder 268. Based on the signals from the encoder 268, the control circuit may calculate the stage of deployment of the knife 32 in the end effector 12. That is, the control circuit can calculate if the knife 32 is fully deployed, fully retracted, or at an intermittent stage. Based on the calculation of the stage of deployment of the end effector 12, the control circuit may send a signal to the second motor 265 to control its rotation to thereby control the reciprocating movement of the threaded rod 266.

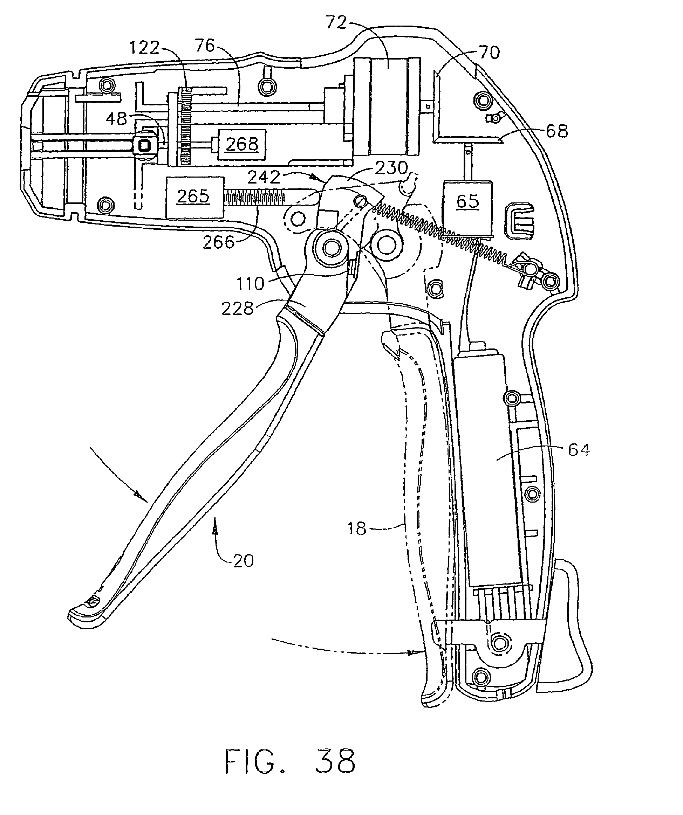

In operation, as shown in Figure 37, when the closure trigger 18 is not locked into the clamped position, the firing trigger 20 rotated away from the pistol grip portion 26 of the handle 6 such that the front face 242 of the upper portion 230 of the firing trigger 20 is not in contact with the proximate end of the threaded rod 266. When the operator retracts the closure trigger 18 and locks it in the clamped position, the firing trigger 20 rotates slightly towards the closure trigger 18 so that the operator can grasp the firing trigger 20, as shown in Figure 38. In this position, the front face 242 of the upper portion 230 contacts the proximate end of the threaded rod 266.

As the user then retracts the firing trigger 20, after an initial rotational amount (e.g., 5 degrees of rotation) the run motor sensor 110 may be activated such that, as explained above, the sensor 110 sends a signal to the motor 65 to cause it to rotate at a forward speed proportional to the amount of retraction force applied by the operator to the firing trigger 20. Forward rotation of the motor 65 causes the main drive shaft 48 to rotate via the gear drive train, which causes the knife 32 and sled 33 to travel down the channel 22 and sever tissue clamped in the end effector 12. The control circuit receives the output signals from the encoder 268 regarding the incremental rotations of the main drive shaft assembly and sends a signal to the second motor 265 to cause the second motor 265 to rotate, which causes the threaded rod 266 to retract into the motor 265. This allows the upper portion 230 of the firing trigger 20 to rotate CCW, which allows the lower portion 228 of the firing trigger to also rotate CCW. In that way, because the reciprocating movement of the threaded rod 266 is related to the rotations of the main drive shaft assembly, the operator of the instrument 10, by way of his/her grip on the firing trigger 20, experiences tactile feedback as to the position of the end effector 12. The retraction force applied by the operator, however, does not directly affect the drive of the main drive shaft assembly because the firing trigger 20 is not geared into the gear drive train in this embodiment.

By virtue of tracking the incremental rotations of the main drive shaft assembly via the output signals from the encoder 268, the control circuit can calculate when the knife 32 is fully deployed (i.e., fully extended). At this point, the control circuit may send a signal to the motor 65 to reverse direction to cause retraction of the knife 32. The reverse direction of the motor 65 causes the rotation of the main drive shaft assembly to reverse direction, which is also detected by the encoder 268. Based on the reverse rotation detected by the encoder 268, the control circuit sends a signal to the second motor 265 to cause it to reverse rotational direction such that the threaded rod 266 starts to extend longitudinally from the motor 265. This motion forces the upper portion 230 of the firing trigger 20 to rotate CW, which causes the lower portion 228 to rotate CW. In that way, the operator may experience a CW force from the firing trigger 20, which provides feedback to the operator as to the retraction position of the knife 32 in the end effector 12. The control circuit can determine when the knife 32 is fully retracted. At this point, the control circuit may send a signal to the motor 65 to stop rotation.

According to other embodiments, rather than having the control circuit determine the position of the knife 32, reverse motor and stop motor sensors may be used, as described above. In addition, rather than using a proportional sensor 110 to control the rotation of the motor 65, an on/off switch or sensor can be used. In such an embodiment, the operator would not be able to control the rate of rotation of the motor 65. Rather, it would rotate at a preprogrammed rate.

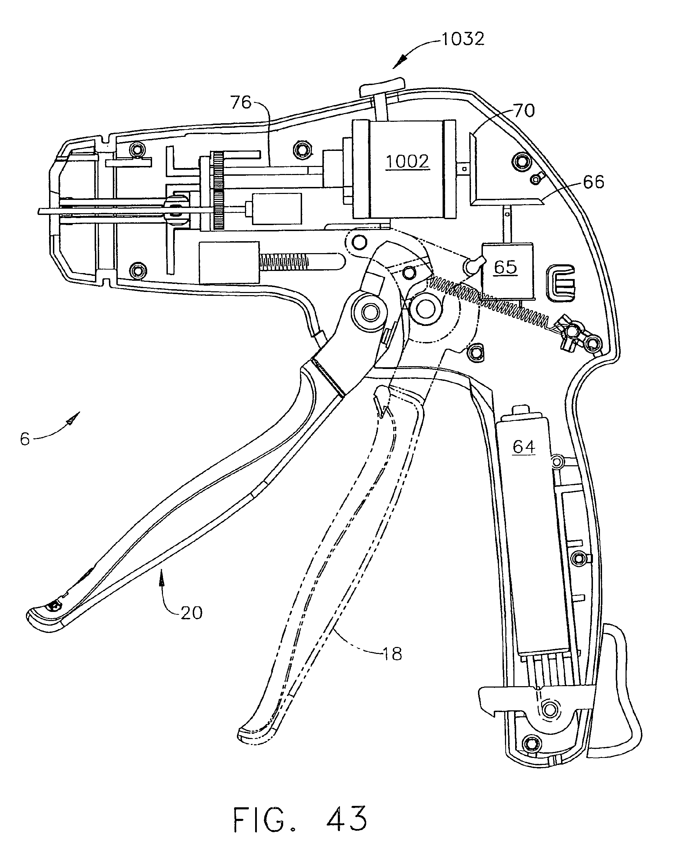

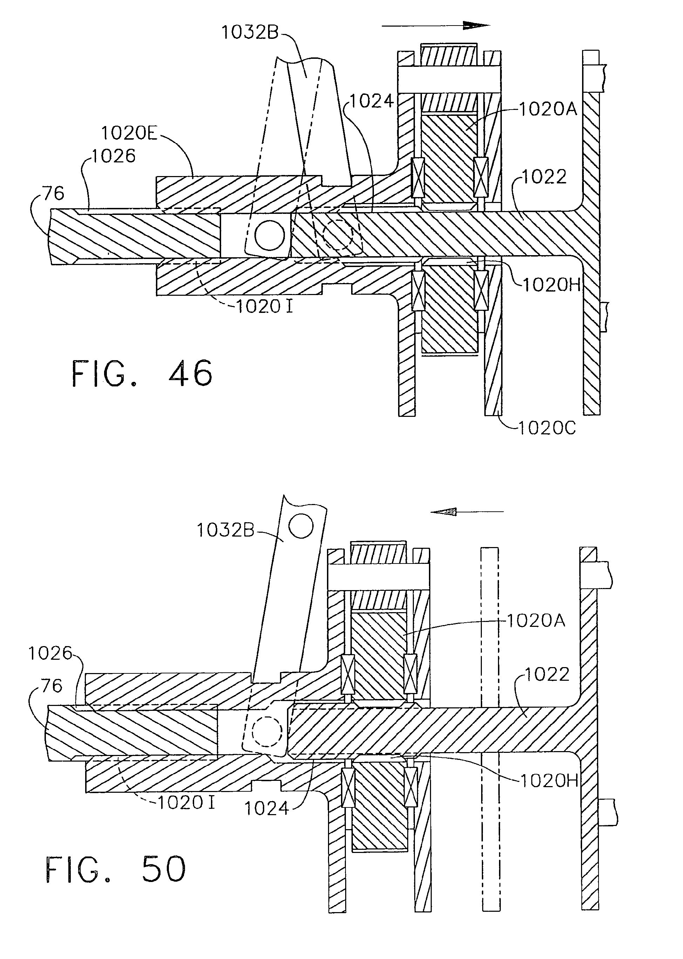

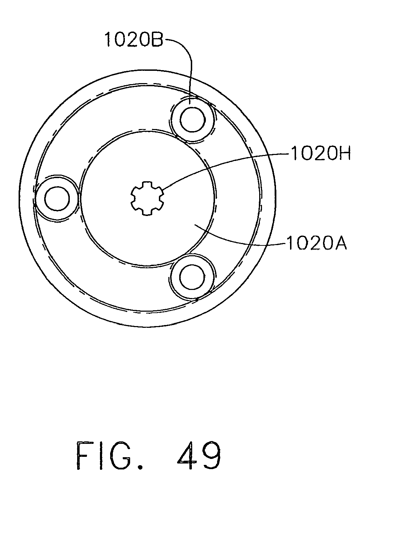

With general reference to Figures 43 through 50, in various embodiments of the invention, a gear shifting assembly 1002 may be employed for operative interaction with the motor 65, for example, of the surgical instrument 10. The gear shifting assembly 1002 can be connected to the motor 65 and to the drive shaft 76 and can be configured to permit a user to adjust mechanical power transferred to the drive shaft 76 from the motor 65. As described below in more detail, the gear shifting assembly 1002 allows for the selective increase or decrease of gear ratio for transfer of power developed by the motor 65 of the instrument 10. This selective increase/decrease feature can be beneficial for use in association with surgical operations that involve using the instrument 10 to cut/staple various types and densities of tissue.

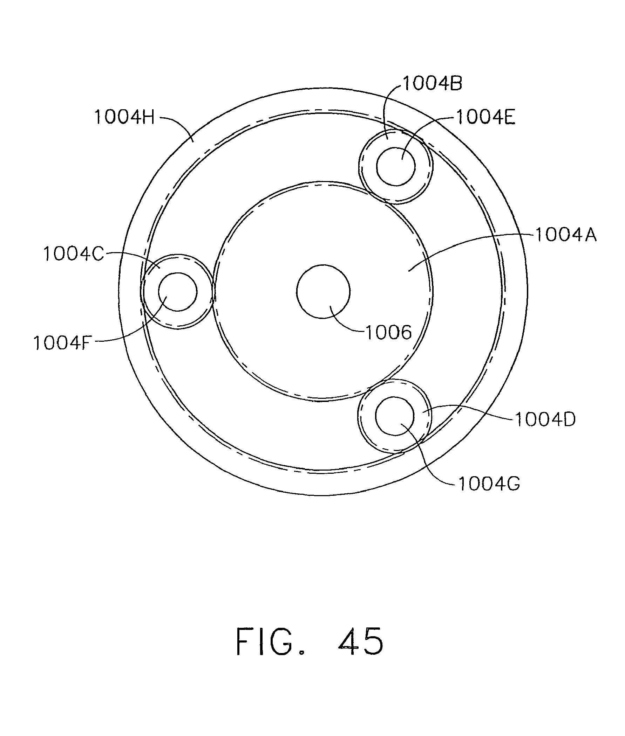

With reference to Figures 43 through 47, the gear shifting assembly 1002 includes a first stage gear assembly 1004 receiving mechanical input power from an input shaft 1006 connected to the motor 65. In various embodiments, the input shaft 1006 may connected directly to the motor 65, or power may be transferred from the motor 65 to the input shaft 1006 through one or more other components, such as the bevel gear assemblies 66, 70, for example. As shown more particularly in Figure 45, the first stage gear assembly 1004 may include a sun gear 1004A intermeshed at least partially with one or more surrounding planet gears 1004B, 1004C, 1004D to provide a planetary gear arrangement for the first stage gear assembly 1004. During operation of the instrument 10, the sun gear 1004A of the first stage gear assembly 1004 may be connected to the input shaft 1006 for transferring mechanical input power from the motor 65 to cause rotation of the sun gear 1004A. It can be seen that, as a consequence of the rotation of the sun gear 1004A, each of the planet gears 1004B, 1004C, 1004D, also rotate accordingly. Each of the planet gears 1004B, 1004C, 1004D may be connected through pins 1004E, 1004F, 1004G (respectively) to transfer mechanical power generated by the rotational movement of the sun gear 1004A to a gear disc 1004H of the first stage gear assembly 1004, as shown.

The gear disc 1004H of the first stage gear assembly 1004 may be connected to an input shaft 1008 which may be connected, in turn, to a second gear stage assembly 1010. The second stage gear assembly 1010 may be structured in substantial accordance with the structure and components employed by the first stage gear assembly 1004 (described above). The second stage gear assembly 1010 may include a sun gear 1010A intermeshed at least partially with one or more planet gears, such as planet gear 1010B, for example, to provide a planetary gear arrangement for the assembly 1010. The sun gear 1010A of the second stage gear assembly 1010 may be connected to the input shaft 1008 for transferring rotational input power received from the first stage gear assembly 1004. In a fashion similar to the planet gears 1004B, 1004C, 1004D of the first stage gear assembly 1004, the planet gears 1010B may be connected through pins 1010C to transfer power generated by the rotational movement of the sun gear 1010A to a gear disc 1010D of the second stage gear assembly 1010.

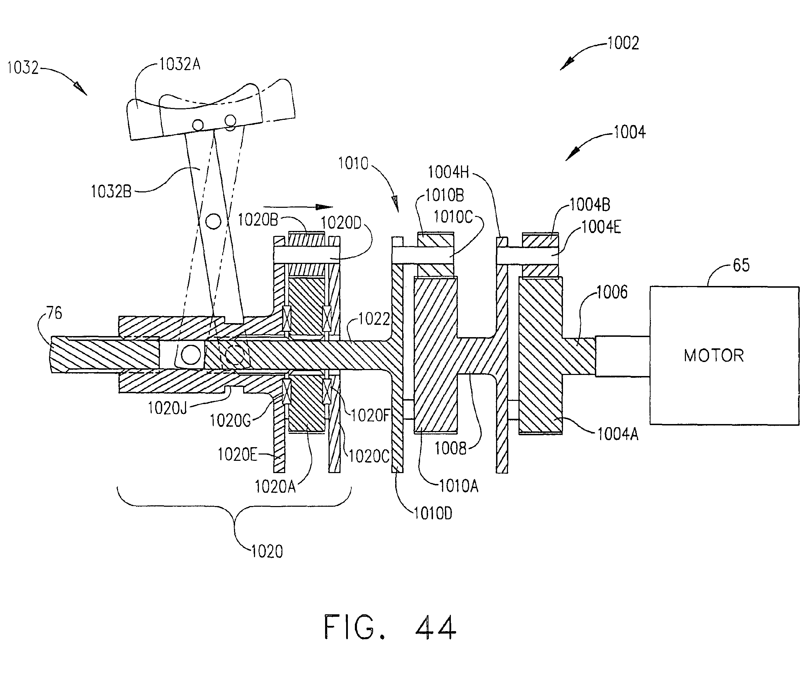

In a first gear setting of the gear shifting assembly 1002, as shown in Figure 44, the first and second stage gear assemblies 1004, 1010 can be coupled to drive shaft 76 of the instrument 10. It can be appreciated, however, that more or less gear assemblies than the gear assemblies 1004, 1010 illustrated, or portions thereof, may be suitably employed in the instrument 10, depending on the gear ratio or application desired for the instrument 10. For example, in certain embodiments, a third stage gear assembly could be included in the drive train with an input shaft connected to the output of the second stage gear assembly 1010.

In various embodiments, a gear coupling assembly 1020 may be connected to the gear disc 1010D of the second stage gear assembly 1010 through an input shaft 1022. The gear coupling assembly 1020 may include a sun gear 1020A at least partially intermeshed with one or more planet gears, such as planet gear 1020B. This planetary gear arrangement, including the sun gear 1020A and planet gear 1020B, may be abutted by a retainer disc 1020C connected through a pin 1020D extending through each of the planet gears 1020B to a collar 1020E. In addition, a thrust bearing 1020F may be positioned between the sun gear 1020A and the retainer disc 1020C; and a thrust bearing 1020G may be positioned between the sun gear 1020A and the collar 1020E, to promote secure positioning of the sun gear 1020A within the gear coupling assembly 1020.

The sun gear 1020A may include a spline section 1020H which can be structured to correspondingly intermesh with a spline section 1024 formed on the input shaft 1022. In the first gear setting illustrated in Figure 44, the spline section 1020H of the sun gear 1020A is not intermeshed with the spline section 1024 of the input shaft 1022. It can be appreciated that the first gear setting provides direct drive from the second stage gear assembly 1010 to the retainer disc 1020C of the gear coupling assembly 1020, without operative interaction of the sun gear 1020A with the input shaft 1022. In other words, the sun gear 1020A of the gear coupling assembly 1020 is permitted to freewheel in the first gear setting and is not coupled to the drive shaft 76 along with the first and second stage gear assemblies 1004, 1010. The collar 1020E includes a spline section 1020I which can be structured to correspondingly intermesh with a spline section 1026 formed on the drive shaft 76. It can be seen that, in the first gear setting, the spline section 1020I of the collar 1020E intermeshes with the spline section 1026 of the drive shaft 76 to transfer mechanical rotational power from the collar 1020E to the drive shaft 76. In addition, in the first gear setting, the spline section 1020I of the collar 1020E may correspondingly intermesh with the spline section 1024 on the input shaft 1022.

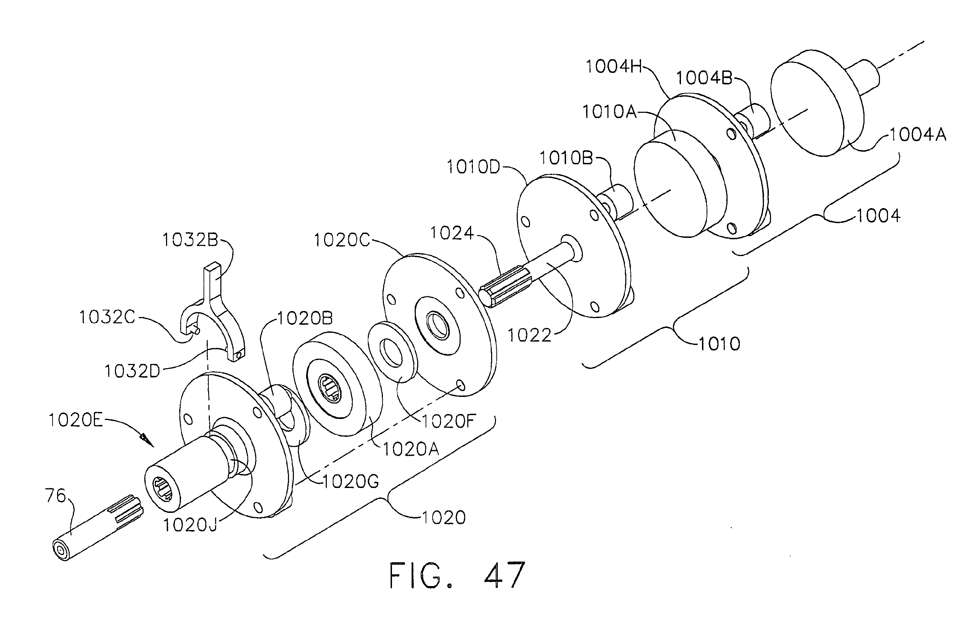

In various embodiments, the gear coupling assembly 1020 may be moved from or into the first gear setting by use of a gear selector assembly 1032. The gear selector assembly 1032 includes a switch 1032A connected to a yoke 1032B. The switch 1032A may be configured to permit the thumb or finger of a user, for example, to move the gear coupling assembly 1020 from or into the first gear setting through its connection to the yoke 1032B. As shown more particularly in Figure 47, the yoke 1032B may be connected to the collar 1020E of the gear coupling assembly 1020 by being received into a yoke receiving groove 1020J positioned around at least a portion of the circumference of the collar 1020E. The yoke 1032B may include one or more pins 1032C, 1032D extending from the yoke 1032B that can be structured to be received into the yoke receiving groove 1020J to promote securement of the yoke 1032B therein. As shown in Figure 44, the gear selector assembly 1032 has been activated to put the gear shifting assembly 1002 in the first gear setting position.

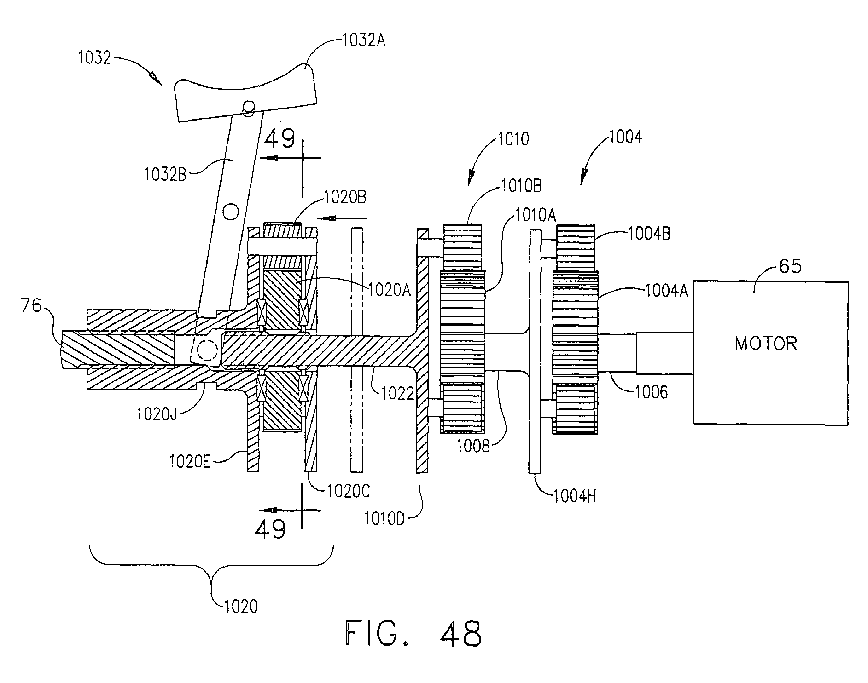

With reference to Figures 48 through 50, in a second gear setting of the gear shifting assembly 1002, the gear coupling assembly 1020 can be selectively moved distally with respect to the motor 65 to engage or couple the spline section 1020H of the sun gear 1020A with the spline section 1024 of the input shaft 1022. The movement of the gear coupling assembly 1020 may be effected by action of the yoke 1032B through its connection to the collar 1020E of the gear coupling assembly 1020. As described above, the action of the yoke 1032B in moving the gear coupling assembly 1020 between first and second gear settings may be effected by a user activating the switch 1032A of the gear selector assembly 1032. It can be seen that the spline section 1020I of the collar 1020E remains engaged or intermeshed with the spline section 1026 formed on the drive shaft 76 in both first and second gear settings to transfer mechanical power through the gear coupling assembly 1020 to the drive shaft 76.

It can be appreciated that in the first gear setting, only the first and second stage gear assemblies 1004, 1010 are operatively involved with the motor 65 in directly driving the drive shaft 76. The first gear setting can be used for comparatively lower torque, higher speed applications of the drive shaft 76, such as for operations involving cutting/stapling relatively low density tissue, for example. In the second gear setting, the planetary gear arrangement of the gear coupling assembly 1020 can be coupled to the drive train to provide comparatively higher torque, lower speed action of the drive shaft 76, such as for operations involving cutting/stapling relatively high density tissue, for example. In general, in various embodiments, the gear shifting assembly 1002 permits a user to achieve an appropriate blend of torque and speed for the drive train, depending on the needs of the various operations in which the instrument 10 is employed on tissue of different density, thickness, or other characteristics.

Although the present invention has been described herein in connection with certain disclosed embodiments, many modifications and variations to those embodiments may be implemented. For example, different types of end effectors may be employed. Also, where materials are disclosed for certain components, other materials may be used. The foregoing description and following claims are intended to cover all such modification and variations.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

## Claims

1. A surgical cutting and fastening instrument comprising:
(a) a drive shaft;
(b) a motor; and
(c) a gear shifting assembly connected to the drive shaft and the motor, the gear shifting assembly comprising:
at least a first stage gear assembly coupled to the motor and to the drive shaft for operating the gear shifting assembly in a first gear setting; and
a gear coupling assembly for selectively coupling at least one additional gear to the drive shaft for operating the gear shifting assembly in a second gear setting.

2. The instrument of Claim 1, wherein the first stage gear assembly comprises a sun gear intermeshed at least partially with one or more planet gears to provide a planetary gear arrangement for the first stage gear assembly.

3. The instrument of Claim 1, further comprising a second stage gear assembly connected to the first stage gear assembly.

4. The instrument of Claim 3, wherein at least one of the first stage gear assembly or the second stage gear assembly comprises a sun gear intermeshed at least partially with one or more planet gears to provide a planetary gear arrangement for the first stage gear assembly or the second stage gear assembly.

5. The instrument of Claim 1, wherein the gear coupling assembly further comprises a sun gear at least partially intermeshed with one or more planet gears to provide a planetary gear arrangement which can be selectively coupled to the first stage gear assembly.

6. The instrument of Claim 5, wherein the sun gear of the gear coupling assembly further includes a spline section structured to correspondingly intermesh in the second gear setting with a spline section formed on an input shaft received from the first stage gear assembly into the gear coupling assembly.

7. The instrument of Claim 5, wherein the sun gear of the gear coupling assembly further includes a spline section structured to not correspondingly intermesh in the first gear setting with a spline section formed on an input shaft received from the first stage gear assembly into the gear coupling assembly.

8. The instrument of Claim 1, the gear coupling assembly further comprising a collar including a spline section formed therein.

9. The instrument of Claim 8, further comprising the spline section of the collar being structured to correspondingly intermesh with a spline section formed on the drive shaft in the first gear setting or the second gear setting.

10. The instrument of Claim 1, wherein the gear shifting assembly further comprises a gear selector assembly for moving the gear coupling assembly between the first and second gear settings.

11. A surgical cutting and fastening instrument comprising:
(a) a drive shaft;
(b) a motor; and
(c) a gear shifting assembly connected to the drive shaft and the motor, the gear shifting assembly comprising:
a first stage gear assembly coupled to the motor and to the drive shaft for operating the gear shifting assembly in a first gear setting, the first stage gear assembly comprising a sun gear intermeshed at least partially with one or more planet gears to provide a planetary gear arrangement for the first stage gear assembly;
a second gear stage assembly connected to the first gear stage assembly and to the drive shaft, the second stage gear assembly comprising a sun gear intermeshed at least partially with one or more planet gears to provide a planetary gear arrangement for the second stage gear assembly;
a gear coupling assembly for selectively coupling at least one additional gear to the second stage gear assembly for operating the gear shifting assembly in a second gear setting, the gear coupling assembly further comprising a sun gear at least partially intermeshed with one or more planet gears to provide a planetary gear arrangement which can be selectively coupled to the first stage gear assembly.

12. A surgical cutting and fastening instrument comprising:
(a) a drive shaft;
(b) a motor; and
(c) a gear shifting assembly connected to the drive shaft and the motor, the gear shifting assembly comprising:
a first stage gear assembly coupled to the motor and to the drive shaft for operating the gear shifting assembly in a first gear setting, the first stage gear assembly comprising a sun gear intermeshed at least partially with one or more planet gears to provide a planetary gear arrangement for the first stage gear assembly;
a second gear stage assembly connected to the first gear stage assembly and to the drive shaft, the second stage gear assembly comprising a sun gear intermeshed at least partially with one or more planet gears to provide a planetary gear arrangement for the second stage gear assembly;
a gear coupling assembly for selectively coupling at least one additional gear to the second stage gear assembly for operating the gear shifting assembly in a second gear setting, the gear coupling assembly further comprising a sun gear at least partially intermeshed with one or more planet gears to provide a planetary gear arrangement which can be selectively coupled to the second stage gear assembly; and,
wherein the sun gear of the gear coupling assembly further includes a spline section structured to correspondingly intermesh in the second gear setting with a spline section formed on an input shaft received into the gear coupling assembly from the second stage gear assembly, and the spline section of the sun gear of the gear coupling assembly further being structured to not correspondingly intermesh in the first gear setting with a spline section formed on an input shaft received into the gear coupling assembly from the second stage gear assembly.
